# EUROPÄISCHE PATENTSCHRIFT

(11) **EP 1 977 655 B1**
(45) Veröffentlichungstag und Bekanntmachung des Hinweises auf die Patenterteilung: **18.05.2011**
(21) Anmeldenummer: 08153113.9
(22) Anmeldetag: 20.03.2008
(51) Int. Cl.: A23L 1/22, C07C 49/825, C07C 49/835, C07C 49/84

(54) **Aromakompositionen von Alkamiden mit Hesperetin und/oder 4-Hydroxydihydrochalkonen und deren Salzen zur Verstärkung süßer sensorischer Eindrücke**
Aroma compositions of alkamides with hesperetin and/or 4-hydroxydihydrochalkones and their salts for reinforcing sweet sensory impressions
Compositions d'aromes d'alcamides comprenant de l'hespérétine et/ou des 4-hydroxydihydrochalcones et leurs sels destinées à renforcer les impressions sensorielles sucrées

(30) Priorität: 29.03.2007 US 908730 P
(43) Veröffentlichungstag der Anmeldung: 08.10.2008
(73) Patentinhaber: Symrise AG, 37603 Holzminden (DE)
(72) Erfinder: Ley, Jakob, 37603, Holzminden (DE); Langer, Kathrin, 37586, Dassel (DE); Krammer, Gerhard, 37603, Holzminden (DE); Reinders, Gerald, 37671, Höxter (DE)
(74) Vertreter: Eisenführ, Speiser & Partner

(56) Entgegenhaltungen:
- EP-A- 1 258 200
- EP-A- 1 642 886
- WO-A-2007/014879
- JP-A- 10 276 712

## Beschreibung

Die Erfindung betrifft primär Aromakompositionen von (i) bestimmten speichelfördernden, prickelnd, scharf und/oder warm schmeckenden Alkamiden (wie z.B. Pellitorinen, Spilanthol) mit (ii) Hesperetin (5,7-Dihydroxy-3-(3-hydroxy-4-methoxyphenyl)-chroman-4-on) bzw. dessen Enantiomeren und/oder Salzen und/oder (iii) 4-Hydroxydihydrochalkonen (3-(4-Hydroxyphenyl)-1-phenylpropan-1-onen) und/oder deren Salzen, deren Verwendung zur Verstärkung des süßen Geschmacks süß schmeckender Stoffe oder des süßen Geruchseindrucks von Aromastoffen, die ein süßen Geruchseindruck verursachen, insbesondere aber die Verstärkung des süßen Anfangsgeschmacks bzw. -geruchs (Anfangssüße). Die Erfindung betrifft somit die Verwendung der besagten Aromakompositionen als genereller Süßverstärker und Verstärker der Anfangssüße. Ferner betrifft die Erfindung bestimmte Zubereitungen, die einen wirksamen Gehalt an den besagten Aromakompositionen von (i) Alkamiden (wie insbesondere Pellitorinen, Spilanthol) mit (ii) Hesperetin bzw. dessen Enantiomeren und/oder Salzen und/oder (iii) 4-Hydroxydihydrochalkonen und/oder deren Salzen enthalten sowie Verfahren zum Verstärken des süßen Geschmacks bzw. der Anfangssüße eines süß schmeckenden Stoffes oder des süßen Geruchseindrucks bzw. der Anfangssüße eines Aromastoffes, der einen süßen Geruchseindruck verursacht. Weiterhin betrifft die Erfindung die Verwendung von bestimmten Alkamiden (i) zur Verstärkung der Anfangssüße einer Zubereitung umfassend (ii) Hesperetin bzw. dessen Enantiomere und/oder Salze und/oder (iii) 4-Hydroxydihydrochalkone und/oder deren Salze sowie (b) süß schmeckende Stoffe und/oder (c) Aromastoffe, die einen süßen Geruchseindruck verursachen.

Nahrungs- oder Genussmittel, die einen hohen Zuckergehalt (vor allem Sucrose (= Saccharose), Lactose, Glucose oder Fructose oder deren Mischungen) aufweisen, werden in der Regel von Verbrauchern auf Grund der Süße stark präferiert. Auf der anderen Seite ist allgemein bekannt, dass ein hoher Gehalt an leicht verstoffwechselbaren Kohlenhydraten den Blutzuckerspiegel stark ansteigen lässt, zur Bildung von Fettdepots führt und letztendlich zu gesundheitlichen Problemen wie Übergewicht, Fettleibigkeit, Insulinresistenz, Altersdiabetes und deren Folgeerkrankungen führen kann. Insbesondere kommt erschwerend hinzu, dass viele der oben genannten Kohlenhydrate zusätzlich die Zahngesundheit beeinträchtigen können, da sie von bestimmten Bakteriensorten in der Mundhöhle zu beispielsweise Milchsäure abgebaut werden und den Zahnschmelz der juvenilen oder adulten Zähne angreifen können (Karies).

Daher ist es schon lange ein Ziel, den Zuckergehalt von Nahrungs- und/oder Genussmitteln auf das unbedingt nötige Maß oder darunter zu reduzieren. Eine entsprechende Maßnahme besteht im Einsatz von Süßstoffen: das sind chemisch einheitliche Substanzen, die selbst keinen oder nur einen sehr geringen kalorischen Brennwert haben und gleichzeitig einen starken süßen Geschmackseindruck verursachen; die Stoffe sind in der Regel nicht-kariogen (eine Übersicht ist z.B. zu finden in Journal of the American Dietetic Association 2004, 104 (2), 255-275). Die sogenannten Bulk-Süßstoffe wie Sorbitol, Mannitol oder andere Zuckeralkohole sind zwar teilweise ausgezeichnete Süßungsmittel und können auch die übrigen nahrungsmitteltechnischen Eigenschaften von Zuckern teilweise ersetzen, führen aber bei zu häufiger Aufnahme bei einem Anteil der Bevölkerung zu osmotisch bedingten Verdauungsproblemen. Die nicht-nutritiven, hochintensiven Süßstoffe sind zwar durch ihre geringe Einsatzkonzentration gut geeignet, Süße in Nahrungsmittel zu bringen, zeigen jedoch oft geschmackliche Probleme durch im Vergleich zu Zucker unähnliche Zeit-Intensitätsprofile (z.B. Sucralose, Stevioside, Cyclamat), einen bitteren und/oder adstringierenden Nachgeschmack (z.B. Acesulfam K, Saccharin), ausgeprägte zusätzliche Aromaeindrücke (z.B. Glycerrhyzinsäureammoniumsalz). Einige der Süßstoffe sind gegenüber Hitze nicht besonders stabil (z.B. Thaumatin, Brazzein, Monellin), sind nicht in allen Anwendungen stabil (z.B. Aspartam) und sind teilweise sehr langanhaltend in ihrer süßen Wirkung (starker süßer Nachgeschmack, z.B. Saccharin).

Eine Verbesserung der geschmacklichen Eigenschaften, insbesondere des Nachgeschmack-Problems von nicht-nutritiven, hochintensiven Süßstoffen kann durch den Einsatz von Tanninsäure, z.B. wie in WO 98/20753 beschrieben, oder Phenolsäuren wie in US 3,924,017 erreicht werden. Allerdings sind solche Stoffe auf Grund ihrer Catechol-Einheiten nicht besonders stabil in Anwendungen.

Eine andere Möglichkeit - ohne den Einsatz von nicht-nutritiven Süßstoffen - besteht darin, den Zuckergehalt von Nahrungs- und/oder Genussmitteln zu senken und sensorisch schwach oder nicht wahrnehmbare Stoffe zuzusetzen, die die Süße mittelbar oder unmittelbar verstärken, wie z.B. in WO 2005/041684 beschrieben. Die in WO 2005/041684 beschriebenen Stoffe sind aber ausdrücklich nicht-natürlichen Ursprungs und somit aus toxikologischer Sicht schwieriger zu bewerten als Stoffe natürlicher Herkunft, insbesondere wenn letztere in Nahrungs- oder Genussmitteln vorkommen oder aus Rohstoffen zur Nahrungs- oder Genussmittelgewinnung stammen. In EP 1 291 342 werden solche Stoffe natürlicher Herkunft (Pyridinium-Betaine) beschrieben; allerdings wird durch sie nicht selektiv der süße Geschmack, sondern es werden auch andere Geschmacksrichtungen wie Umami oder Salzigkeit beeinflusst. Zudem sind die offenbarten Stoffe nur mit hohem Aufwand zu reinigen.

In WO 2007/014879 (Symrise) wird der Einsatz von Hesperetin als Verstärker des süßen Geschmacks von Zucker-reduzierten, der Ernährung oder dem Genuss dienenden Zubereitungen empfohlen. Bisweilen nachteilig ist allerdings bei Einsatz von Hesperetin dessen geringe Löslichkeit insbesondere in klaren, wässrigen Anwendungen (wie z.B. klaren Cola- oder Zitronenlimonaden) sowie die vergleichsweise schwach ausgeprägte Süßverstärkung in sauren Nahrungs- und Genussmitteln. In WO 2007/014879 wird die Verwendung von Hesperetin auch in Kombination mit 4-Hydroxydihydrochalkonen beschrieben. Dabei können schon erhebliche Reduzierungen der Zucker- oder Süßstoffmengen bei gleicher Süße erreicht werden, allerdings wird das Profil, insbesondere die Anfangssüße, gelegentlich als nicht ausreichend bzw. zufriedenstellend wahrgenommen.

Daher ist es wünschenswert, Stoffe oder Stoffmischungen (Aromakompositionen) zu finden, die in geringen Konzentrationen süße Geschmackseindrücke von süßen Stoffen, bevorzugt den süßen Geschmackseindruck von Zucker-reduzierten Nahrungs- und Genussmitteln, insbesondere von Zucker-reduzierten sauren und/oder klaren wässrigen Nahrungs- und Genussmitteln wirkungsvoll verstärken, ohne das übrige Aromaprofil negativ zu beeinflussen. Ebenso ist es wünschenswert, Stoffe zu finden, die in geringen Konzentrationen süße Geruchseindrücke von Aromastoffen, die einen süßen Geruchseindruck verursachen, wirkungsvoll verstärken.

Es war die primäre Aufgabe der vorliegenden Erfindung, Aromakompositionen zu finden, die (a) selektiv zur Verstärkung des süßen Geschmacks eines süß schmeckenden Stoffes und/oder des süßen Geruchseindrucks eines Aromastoffes, der einen süßen Geruchseindruck verursacht, geeignet sind, vorzugsweise ohne das übrige Aromaprofil negativ zu beeinflussen und (b) besonders geeignet sind, die Anfangssüße der Anwendung zu verstärken.

Gemäß einem ersten Aspekt der vorliegenden Erfindung wird die gestellte Aufgabe gelöst durch eine Aromakomposition, bestehend aus oder enthaltend eine Substanz (i) sowie entweder
- eine Substanz (ii) oder
- eine Substanz (iii) oder
- eine Mischung von Substanzen (ii) und (iii), wobei gilt:
   (i) besteht aus einem oder mehreren Alkamiden ausgewählt aus der Gruppe bestehend aus:
      2E,4E-Decadiensäure-N-isobutylamid (Pellitorin), 2E,4Z- Decadiensäure-N-isobutylamid (cis-Pellitorin), 22,4Z- Decadiensäure-N-isobutylamid, 2Z,4E- Decadiensäure-N-isobutylamid, 2E,4E-Decadiensäure-N-([2S]-2-methylbutyl)amid, 2E,4E-Decadiensäure-N-([2S]-2-methylbutyl)amid, 2E,4E-Decadiensäure-N-([2R]-2-methylbutylamid), 2E,4Z-Decadiensäure-N-(2-methylbutyl)amid, 2E,4E-Decadiensäure-N-piperid (Achilleamid), 2E,4E-Decadiensäure-N-piperid (Sarmentin), 2E-Decensäure-N-isobutylamid, 3E-Decensäure-N-isobutylamid, 3E-Nonensäure-N-isobutylamid, 2E,6Z,8E-Decatriensäure-N-isobutylamid (Spilanthol), 2E,6Z,8E-Decatriensäure-N-([2S]-2-methylbutyl)amid (Homospilanthol), 2E,6Z,8E-Decatriensäure-N-([2R]-2-methylbutyl)amid, 2E-Decen-4-insäure-N-isobutylamid, 2Z-Decen-4-insäure-N-isobutylamid, 2E,6Z,8E,1 OE-Dodecatetraensäure-N-(2-methylpropyl)amid (α-Sanshool), 2E,6Z,8E,10E-Dodecatetraensäure-N-(2-hydroxy-2-methylpropyl)amid (α-Hydroxysanshool), 2E,6E,8E,10E-Dodecatetraensäure-N-(2-hydroxy-2-methylpropyl)amid (β-Hydroxysanshool), 2E,4E,8Z,10E,12E-Tetradecapentaensäure-N-(2-hydroxy-2-methylpropyl)amid (γ-Hydroxysanshool), 2E,4E,8E,10E,12E-Tetradecapentaensäure-N-(2-hydroxy-2-methylpropyl)amid (γ-Hydroxyisosanshool), 2E,4E,8Z,10E,12E-Tetradecapentaensäure-N-(2-methyl-2-propenyl)amid (γ-dehydrosanshool), 2E,4E,8Z,10E,12E-Tetradecapentaensäure-N-(2-methylpropyl)amid (γ-Sanshool), 2E,4E,8Z,11Z-Tetradecatetraensäure-N-(2-hydroxy-2-methylpropyl)amid (Bungeanool), 2E,4E,8Z,11 E-Tetradecatetraensäure-N-(2-hydroxy-2-methylpropyl)amid (Isobungeanool), 2E,4E,8Z-Tetradecatriensäure-N-(2-hydroxy-2-methylpropyl)amid (Dihydrobungeanool) und 2E,4E -Tetradecadiensäure-N-(2-hydroxy-2-methylpropyl)amid (Tetrahydrobungeanool);
   (ii) ist Hesperetin der Formel (I) 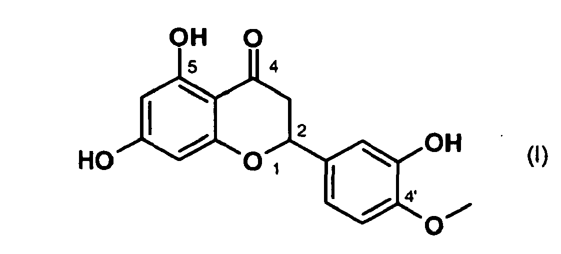 wobei das Hesperetin der Formel (I) als (2*S*)-Enantiomer, (2*R*)-Enantiomer oder beliebige Mischung der beiden Enantiomere vorliegt, und/oder dessen Salzen
      und
   (iii) ist
      - ein 4-Hydroxydihydrochalkon der Formel (II) 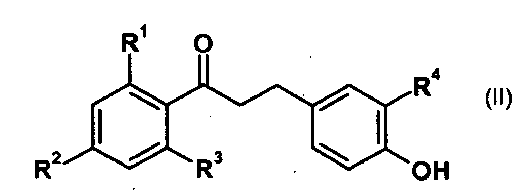 wobei
         R¹, R², R³ und R⁴ unabhängig voneinander jeweils H, OH oder O-Alkyl bedeuten, mit der Maßgabe, dass mindestens einer der Reste R¹, R² oder R³ OH bedeutet,
      - ein Salz eines solchen 4-Hydroxydihydrochalkons der Formel (II),
      - eine Mischung umfassend oder bestehend aus zwei oder mehr unterschiedlichen 4-Hydroxydihydrochalkonen der Formel (II), wobei R¹, R², R³ und R⁴ jeweils die oben angegebene Bedeutung haben,
      - eine Mischung umfassend oder bestehend aus Salzen von zwei oder mehr unterschiedlichen 4-Hydroxydihydrochalkonen der Formel (II), wobei R¹, R², R³ und R⁴ jeweils die oben angegebene Bedeutung haben
         oder
      - eine Mischung umfassend oder bestehend aus einem 4-Hydroxydihydrochalkon der Formel (II) oder zwei oder mehr unterschiedlichen 4-Hydroxydihydrochalkonen der Formel (II), wobei R¹, R², R³ und R⁴ jeweils die oben angegebene Bedeutung haben, und
einem Salz eines 4-Hydroxydihydrochalkons der Formel (II) oder zwei oder mehr Salzen von unterschiedlichen 4-Hydroxydihydrochalkonen der Formel (II), wobei R¹, R², R³ und R⁴ jeweils die oben angegebene Bedeutung haben.
Sofern in einer erfindungsgemäßen Aromakomposition Substanz (ii) vorhanden ist, kann Substanz (iii) abwesend sein, und umgekehrt.

In einer erfindungsgemäßen Aromakomposition, insbesondere in einer der nachfolgend als bevorzugt gekennzeichneten Ausgestaltungen, ist der Einsatz von Hesperetin gegenüber dem Einsatz von Salzen des Hesperetins bevorzugt.

Eine erfindungsgemäße Aromakomposition wird insbesondere vorteilhafterweise verwendet zur Verstärkung des süßen Geschmacks (insbesondere der Anfangssüße) eines süß schmeckenden Stoffes oder des süßen Geruchseindrucks (insbesondere der Anfangssüße) eines Aromastoffes, der einen süßen Geruchseindruck verursacht.

Ganz besonders vorteilhaft ist die Verwendung einer erfindungsgemäßen Aromakomposition zur Verstärkung der Anfangssüße eines süß schmeckendes Stoffes oder eines Aromastoffes, der einen süßen Geruchseindruck verursacht.

Bevorzugt ist eine erfindungsgemäße Aromakomposition, wobei gilt:
(i) besteht aus einem oder mehreren Alkamiden ausgewählt aus der Gruppe bestehend aus:
   2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,4E-Decadiensäure-N-piperid (Achilleamid), 2E,6Z,8E-Decatriensäure-N-isobutylamid (Spilanthol), 2E,6Z,8E-Decatriensäure-N-([2S]-2-methylbutyl)amid (Homospilanthol), 2E,6Z,8E-Decatriensäure-N-([2R]-2-methylbutyl)amid, 2E,6Z,8E,10E-Dodecatetraensäure-N-(2-methylpropyl)amid (α-Sanshool), 2E,6Z,8E,10E-Dodecatetraensäure-N-(2-hydroxy-2-methylpropyl)amid (α-Hydroxysanshool), 2E,4E,8Z,10E,12E-Tetradecapentaensäure-N-(2-methylpropyl)amid (γ-Sanshool) und 2E,4E,8Z,11Z-Tetradecatetraensäure-N-(2-hydroxy-2-methylpropyl)amid (Bungeanool);
   und/oder
(ii) ist Hesperetin der Formel (I) 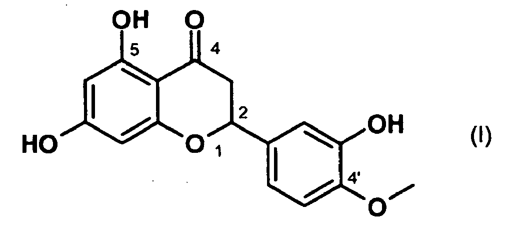 wobei das Hesperetin der Formel (I) als (2*S*)-Enantiomer, (2*R*)-Enantiomer oder beliebige Mischung der beiden Enantiomere vorliegt,
   und/oder
(iii) ist ein 4-Hydroxydihydrochalkon der Formel (II), wobei in der Formel (II) jeweils
   R¹ OH bedeutet
   R² und R³, unabhängig voneinander, H oder OH bedeuten,
      und
   R⁴ H oder Methoxy (OCH₃) bedeutet.

Besonders bevorzugt ist eine erfindungsgemäße Aromakomposition, wobei gilt:
(i) besteht aus einem oder mehreren Alkamiden ausgewählt aus der Gruppe bestehend aus:
   2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,6Z,8E-Decatriensäure-N-isobutylamid (Spilanthol), 2E,6Z,8E,10E-Dodecatetraensäure-N-(2-methylpropyl)amid (α-Sanshool), 2E,4E,8Z,11Z-Tetradecatetraensäure-N-(2-hydroxy-2-methylpropyl)amid (Bungeanool); und/oder
(ii) ist Hesperetin der Formel (I) 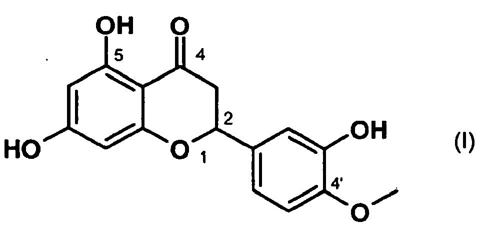 wobei das Hesperetin der Formel (I) als (2*S*)-Enantiomer oder beliebige Mischung der beiden Enantiomere vorliegt,
   und/oder
(iii) besteht aus
   - einem 4-Hydroxydihydrochalkon der Formel (II) ausgewählt aus der Gruppe bestehend aus:
      3-(4-Hydroxyphenyl)-1-(2-hydroxyphenyl)propan-1-on (2',4-Dihydroxydihydrochalkon; Verbindung 1),
      3-(4-Hydroxyphenyl)-1-(2,4-dihydroxyphenyl)propan-1-on (2',4,4'-Trihydroxydihydrochalkon; Davidigenin; Verbindung 2),
      3-(4-Hydroxyphenyl)-1-(2,6-dihydroxyphenyl)propan-1-on (2',4,6'-Trihydroxydihydrochalkon; Verbindung 3),
      3-(4-Hydroxyphenyl)-1-(2,4,6-trihydroxyphenyl)propan-1-on (2',4,4',6'-Tetrahydroxydihydrochalkon; Phloretin; Verbindung 4),
      3-(4-Hydroxy-3-methoxyphenyl)-1-(2-hydroxyphenyl)propan-1-on (2',4-Dihydroxy-3-methoxydihydrochalkon; Verbindung 5),
      3-(4-Hydroxy-3-methoxyphenyl)-1-(2,4-dihydroxyphenyl)propan-1-on (2',4,4'-Trihydroxy-3-methoxydihydrochalkon; Verbindung 6),
      3-(4-Hydroxy-3-methoxyphenyl)-1-(2,6-dihydroxyphenyl)propan-1-on (2',4,6'-Trihydroxy-3-methoxydihydrochalkon; Verbindung 7),
      3-(4-Hydroxy-3-methoxyphenyl)-1-(2,4,6-trihydroxyphenyl)propan-1-on (2',4,4',6'-Tetrahydroxy-3-methoxydihydrochalkon; Verbindung 8) und
      3-(3,4-Dihydroxyphenyl)-1-(2,4,6-trihydroxyphenyl)propan-1-on (2',3,4,4',6'-Tetrahydroxydihydrochalkon; Verbindung 9),
   - einem Salz eines solchen 4-Hydroxydihydrochalkons der Formel (II),
   - einer Mischung umfassend oder bestehend aus zwei oder mehr aus der besagten Gruppe ausgewählten 4-Hydroxydihydrochalkonen der Formel (II),
   - einer Mischung umfassend oder bestehend aus Salzen von zwei oder mehr aus der besagten Gruppe ausgewählten 4-Hydroxydihydrochalkonen der Formel (II)
      oder
   - einer Mischung umfassend oder bestehend aus
einem der aus der besagten Gruppe ausgewählten 4-Hydroxydihydrochalkone der Formel (II) oder zwei oder mehr aus der besagten Gruppe ausgewählten 4-Hydroxydihydrochalkonen der Formel (II) und
einem Salz eines aus der besagten Gruppe ausgewählten 4-Hydroxydihydrochalkons der Formel (II) oder zwei oder mehr Salzen von unterschiedlichen aus der besagten Gruppe ausgewählten 4-Hydroxydihydrochalkonen der Formel (II).

Ganz besonders bevorzugt ist eine erfindungsgemäße Aromakomposition, wobei gilt:
(i) besteht aus einem oder mehreren Alkamiden ausgewählt aus der Gruppe bestehend aus:
   2E,4E-Decadiensäure-N-isobutylamid (Pellitorin), 2E,6Z,8E-Decatriensäure-N-isobutylamid (Spilanthol), 2E,6Z,8E,10E-Dodecatetraensäure-N-(2-methylpropyl)amid (α-Sanshool) und 2E,4E,8Z,11Z-Tetradecatetraensäure-N-(2-hydroxy-2-methylpropyl)amid (Bungeanool);
   und/oder
(ii) ist Hesperetin der Formel (I) 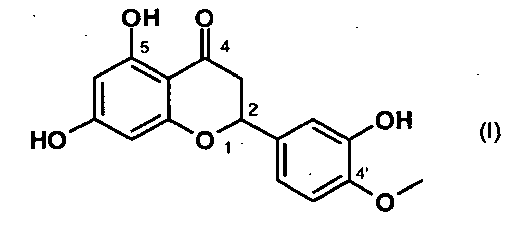 wobei das Hesperetin der Formel (I) als (2*S*)-Enantiomer oder beliebige Mischung der beiden Enantiomere vorliegt,
   und/oder
(iii) besteht aus oder umfasst Phloretin (Verbindung 4).

In erfindungsgemäß zu verwendenden Salzen des Hesperetins der obigen Formel (I) bzw. des 4-Hydroxydihydrochalkons der obigen Formel (II) sind eine, mehrere oder sämtliche deprotonierbaren Gruppen des Hesperetins bzw. des 4-Hydroxydihydrochalkons deprotoniert. Es liegt dann eine entsprechende Menge von Gegenkationen vor, wobei diese vorzugsweise ausgewählt sind aus der Gruppe bestehend aus: einfach positiv geladene Kationen der ersten Haupt- und Nebengruppe, Ammoniumionen, Trialkylammoniumionen, zweifach positiv geladene Kationen der zweiten Haupt- und Nebengruppe sowie dreifach positiv geladene Kationen der 3. Haupt- und Nebengruppe, und deren Mischungen.

Besonders bevorzugte Kationen sind Na⁺, K⁺, NH₄⁺, Ca²⁺, Mg²⁺, Al³⁺ und Zn²⁺.

Die bevorzugten Verbindungen der Formel (II) sind in der folgenden Abbildung noch einmal zur Verdeutlichung aufgeführt (an Verbindung 1 ist exemplarisch das allgemeine Numerierungsschema für Dihydrochalkone angegeben):

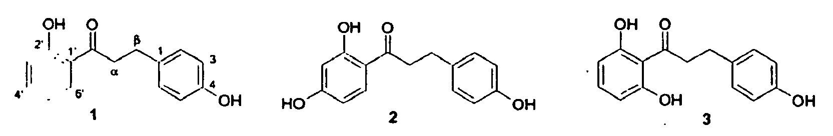

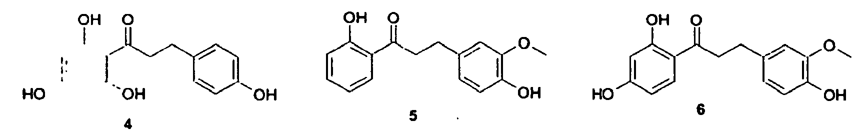

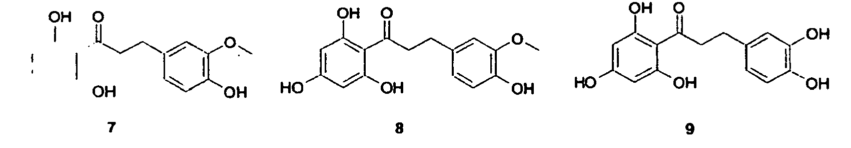

Bevorzugt ist eine erfindungsgemäße Aromakomposition, wobei
- das Gewichtsverhältnis der Gesamtmenge von Substanzen (i) (Alkamide) zu der Gesamtmenge von Substanzen (ii) (Hesperetin) und (iii) (4-Hydroxydihydrochalkonen) in einem Bereich von 1:1000000 bis 1:1 liegt, bevorzugt in einem Bereich von 1:10000 bis 1:10, besonders bevorzugt in einem Bereich von 1:2000 bis 1:50
   und/oder
- das Gewichtsverhältnis der Gesamtmengen von Substanzen (ii) zu Substanzen (iii) im Bereich von 1:10 bis 10:1 liegt, besonders bevorzugt im Bereich von 5:1 bis 1:5 und insbesondere bevorzugt im Bereich von 7:3 bis 3:7.

Vorzugsweise werden die vorstehend angegebenen, erfindungsgemäßen bzw. erfindungsgemäß einzusetzenden Aromakompositionen zur Verstärkung des süßen Geschmacks eines süß schmeckenden Stoffes oder des süßen Geruchseindrucks eines Aromastoffes, der einen süßen Geruchseindruck verursacht, in einer der Ernährung, der Mundpflege oder dem Genuss dienenden Zubereitung eingesetzt, insbesondere zur Verstärkung der Anfangssüße in einer der Ernährung, der Mundpflege oder dem Genuss dienenden Zubereitung.

Die Erfindung beruht auf der überraschenden Erkenntnis, dass die erfindungsgemäßen bzw. erfindungsgemäß zu verwendenden Aromakompositionen nicht nur bereits in sehr geringen Konzentrationen (unter 0,025 Gew.-%, vergleiche hierzu die weiter unten angegebenen Konzentrationsbereiche) den süßen Geschmackseindruck von süß schmeckenden Stoffen (wie weiter unten angegeben), insbesondere aber von Zuckern wie Sucrose, Lactose, Glucose, D-Tagatose und Fructose sowie Zuckeralkoholen wie z.B. Glycerin, Erythritol, Threitol, Arabitol, Ribitol, Xylitol, Sorbitol, Mannitol, Dulcitol, Lactitol überproportional (d.h. synergistisch) steigern und es somit möglich ist, den Zuckergehalt in entsprechenden Nahrungs- und Genussmitteln zu senken, ohne gleichzeitig den süßen Geschmackseindruck zu vermindern, sondern auch den Zeit-Süße-Verlauf der zucker-reduzierten Anwendung positiv beeinflussen, insbesondere die sogenannte Anfangssüße steigern. In niedrigen Konzentrationen (vergleiche hierzu die bevorzugten Einsatzkonzentrationen weiter unten) zeigen die erfindungsgemäßen bzw. erfindungsgemäß zu verwendenden Aromakompositionen einen nur sehr schwachen Eigengeschmack.

JP 2006-296356 beschreibt den Einsatz des Alkamids Spilanthol zur Verstärkung des allgemeinen Aromaeindrucks von Nahrungsmitteln, wobei als Vorteil die geringe Beeinflussung des Geschmackswerts genannt wird. Es wird keine Verstärkung der Süße durch die Anwesenheit des Alkamids, insbesondere keine Verstärkung der Anfangssü-ße beschrieben. Auch eine Kombination des Alkamids mit Hesperetin oder einem 4-Hydroxydihydrochalkon ist nicht offenbart. Besonders vorteilhaft ist, dass die erfindungsgemäßen Aromakompositionen zudem einen reduzierenden oder maskierenden Effekt gegen als bitter, adstringierend, kalkig oder trocken beschriebene unangenehme oder Nach- und/oder Nebengeschmacksnoten zeigen.

Die Erfindung betrifft neben den bislang erläuterten bestimmten Aromakompositionen und deren Verwendung gemäß einem weiteren Aspekt auch entsprechende Zubereitungen, in denen die besagten Aromakompositionen in der erfindungsgemäßen Weise eingesetzt sind.

Eine erfindungsgemäße Zubereitung ist dabei vorzugsweise aus der Gruppe ausgewählt, die aus der Ernährung, der Mundpflege oder dem Genuss dienenden Zubereitungen, Halbfertigwaren, Riech-, Aroma- oder Geschmackstoffkompositionen oder Würzmischungen besteht. Eine erfindungsgemäße Zubereitung umfasst die folgenden Komponenten:
(a) eine erfindungsgemäße Aromakomposition aus speichelfördernden, prickelnd, scharf und/oder warm schmeckenden Alkamiden, Hesperetin und/oder 4-Hydroxydihydrochalkonen wie weiter oben beschrieben sowie zusätzlich
(b) einen oder mehrere süß schmeckende Stoffe
   und/oder
(c) einen oder mehrere Aromastoffe, die einen süßen Geruchseindruck verursachen,
wobei die Gesamtmenge an Komponente (a) in der Zubereitung ausreicht, den süßen Geschmackseindruck (insbesondere die Anfangssüße) des bzw. der süß schmeckenden Stoffe (b) bzw. den süßen Geruchseindruck (insbesondere die Anfangssüße) des bzw. der Aromastoffe (c), die einen süßen Geruchseindruck verursachen, sensorisch zu verstärken, vorzugsweise überproportional (d.h. über eine Wirkung hinaus, die durch Eigensüße verursacht wird).

Hinsichtlich der bevorzugten Bedeutung der Gruppen R¹, R², R³ und R⁴ in Formel (II) gilt dabei das hinsichtlich der erfindungsgemäßen Verwendung gesagte entsprechend.

Auch in den erfindungsgemäßen Zubereitungen ist die Anwesenheit einer Aromakomposition bevorzugt bestehend aus (i) ein oder mehreren speichelfördernden, prickelnd, scharf und/oder warm schmeckenden Alkamiden ausgewählt aus der Gruppe 2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,6Z,8E-Decatriensäure-N-isobutylamid (Spilanthol), 2E,6Z,8E,10E-Dodecatetraensäure-N-(2-methylpropyl)amid (α-Sanshool) und 2E,4E,8Z,11Z-Tetradecatetraensäure-N-(2-hydroxy-2-methylpropyl)amid (Bungeanool) sowie (ii) Hesperetin ([S]- oder [R]-Enantiomer oder Mischungen derselben) und/oder (iii) einem 4-Hydroxydihydrochalkon der Formel (II) ausgewählt aus der Gruppe bestehend aus den oben angegebenen Verbindungen 1 bis 9, einem entsprechenden Salz oder einer entsprechenden Mischung (wie oben im Detail angegeben).

Es sei an dieser Stelle darauf hingewiesen, dass sämtliche Ausführungen zu bevorzugten Ausgestaltungen einer erfindungsgemäßen Aromakomposition, einer erfindungsgemäßen Verwendung, einer erfindungsgemäßen Zubereitung oder eines erfindungsgemäßen Verfahrens jeweils entsprechend für die anderen Erfindungsaspekte gelten.

Eine bevorzugte erfindungsgemäße Zubereitung umfasst als Komponente (b) einen oder mehrere Zucker, wobei die Gesamtmenge an Komponente (a) in der Zubereitung ausreicht, um im Vergleich mit einer Zubereitung oder Halbfertigware, die bei ansonsten identischer Zusammensetzung keine Komponente (a), aber zumindest die 1,05-fache Menge (vorzugsweise zumindest 1,2-fache, bevorzugt 1,4-fache Menge) an Zucker umfasst, den gleichen oder einen verstärkten Süßeeindruck, insbesondere in der Anfangssüße zu vermitteln. Die Zucker sind dabei vorzugsweise ausgewählt aus der Gruppe bestehend aus: Sucrose, Lactose, Glucose, Fructose und deren Mischungen.

Bezüglich des Vorkommens und der Herstellung der unter (i), (ii) und (iii) genannten Substanzen sei für (i) auf WO 2004/000787, WO 2004/043960 und für (ii) und (iii) auf WO 2007/014879 verwiesen. Die in WO 2004/043960 beschriebenen, bevorzugten Mischungen von Pellitorinen sind im Rahmen der vorliegenden Erfindung ebenfalls bevorzugt.

Insbesondere für die speichelfördernden, prickelnd, scharf und/oder warm schmeckende Alkamide der Gruppe (i) sind auch natürliche Quellen bekannt und anwendbar.

Bevorzugte Quellen sind pflanzliche Extrakte wie beispielsweise Alkamide enthaltender Pfefferextrakt (*Piper ssp.,* insbesondere *P. nigrum, P. hispidum, P. tuberculatum, P. longum, P. arboreum, P. futokadsura, P. guineense, P. sarmentosum* oder *Piper nigrum, Var. muntok, P. aff. pedicellatum*), Extrakte aus Zahnwehgras (englisch "toothache grass", *Ctenium aromaticum*), Extrakte aus Estragon (*Artemisia dracunculus*)*,* Bertramwurzel-Extrakte (*Anacyclus ssp.,* insbesondere *Anacylcus pyrethrum L.*)*,* Sonnenhutextrakte (*Echinaceae ssp.,* z.B. *E. angustifolia*), Extrakte aus Szechuan-Pfeffer (*Zanthoxylum ssp.,* insbesondere *Zanthoxylum piperitum, Z. clava-herculin, Z. bungeanum, Z. zanthoxyloides*), Spilanthesextrakt *(Spilanthes ssp.,* insbesondere *Spilanthes acmella*), Extrakte aus *Acmella ssp.* (z.B. *A.ciliata*), Extrakte aus *Achillea ssp.* (z.B. *Achillea wilsoniana,* Extrakte aus Fagara-Arten *(Fagara zanthoxyloides*), Extrakte aus *Heliopsis ssp.* (z.B. *H. longipes),* Extrakte aus *Cissampelos glaberrima,* Extrakte aus *Dinosperma erythrococca,* Extrakte aus der Rinde von *Esenbeckia alata* und Extrakte aus *Stauranthus perforatus.*

Die pflanzlichen Extrakte können aus den entsprechenden frischen oder getrockneten Pflanzen oder Pflanzenteilen, insbesondere aber aus weißen, grünen oder schwarzen Pfefferkörnern *(P. nigrum),* Stangenpfeffer *(P. longum),* Sonnenhutwurzeln, Bertramswurzel, Szechuan-Peffer, Pflanzenteilen der anderen *Zanthoxylum-Arten,* Pflanzenteilen der *Spilanthes-* oder *Acmella*-Arten, Pflanzenteilen der *Fagara-* oder *Heliopsis-*Arten gewonnen werden. Üblicherweise werden die getrockneten Pflanzenteile (z.B. frische oder getrocknete Wurzeln, Früchte, Samen, Rinde, Holz, Stängel, Blätter oder Blüten[teile]), vorzugsweise in zerkleinerter Form, mit einem für Nahrungs- und Genussmittel geeigneten Lösungsmittel bei Temperaturen von 0°C bis zum Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise mit Wasserdampf behandelt werden, meist bei Drücken von 0,01 mbar bis Normaldruck, und/oder in einem für Nahrungs- und Genussmittel geeigneten Lösungsmittel aufgenommen werden.

Ein für Nahrungs- und Genussmittel zur Extraktion geeignetes Lösungsmittel ist vorzugsweise ausgewählt aus der Gruppe bestehend aus: Wasser, Ethanol, Methanol, Propylenglycol, Glycerin, Aceton, Dichlormethan, Essigsäureethylester, Diethylether, Hexan, Heptan, Triacetin, pflanzliche Öle oder Fette, superkritisches Kohlendioxid und deren Gemische.

Eine bevorzugte erfindungsgemäße Zubereitung (wie vorstehend beschrieben, insbesondere in einer bevorzugten Ausgestaltung) umfasst
(b) einen oder mehrere weitere süß schmeckende Stoffe, wobei der oder die weiteren süß schmeckenden Stoffe ausgewählt sind aus der Gruppe bestehend aus:
   - ein oder mehrere Kohlenhydrate (Zucker) ausgewählt aus der Gruppe bestehend aus Sucrose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrin und pflanzliche Zubereitungen enthaltend einen oder mehrere der genannten Kohlenhydrate, (vorzugsweise in einem Anteil von zumindest 5 Gew. %, bevorzugt zumindest 15 Gew.-%), wobei diese Kohlenhydrate auch als natürliche oder künstlich hergestellte Mischung vorliegen können (z.B. als Honig, Invertzuckersirup, hochangereicherte Fructose-Sirupe aus Maisstärke [High Fructose Corn Sirup])
   - ein oder mehrere Zuckeralkohole ausgewählt aus der Gruppe bestehend aus Glycerin, Erythritol, Threitol, Arabitol, Ribitol, Xylitol, Sorbitol, Mannitol, Maltitol, Isomaltit, Dulcitol und Lactitol,
   - ein oder mehrere Proteine und/oder Aminosäuren aus der Gruppe bestehend aus Miraculin, Monellin, Thaumatin, Curculin, Brazzein, Glycin, D-Leucin, D-Threonin, D-Asparagin, D-Phenylalanin, D-Tryptophan, L-Prolin oder aus natürlichen Quellen gewonnenen Extrakte oder Fraktionen, enthaltend diese Aminosäuren und/oder Proteine
   - ein oder mehrere Süßstoffe aus der Gruppe bestehend aus Magap, Natriumcyclamat, Acesulfam K, Neohesperidindihydrochalkon, Saccharin-Natriumsalz, Aspartam, Superaspartam, Neotam, Alitam, Sucralose, Stevioside, Rebaudioside, Lugduname, Carrelame, Sucrononate, Sucrooctate, Monatin und Phyllodulcin, wobei im Falle der natürlich vorkommenden Süßstoffe auch Extrakte oder angereicherte Fraktionen dieser Extrakte verwendet werden können, z.B. Thaumatococcus-Extrakte (Katemfestaude), Stevia-Extrakte, Citrus-Extrakte, Buddah-Tee-Extrakte
   und deren Mischungen
   und/oder
(c) einen oder mehrere Aromastoffe, die einen süßen Geruchseindruck verursachen, wobei der oder die weiteren Aromastoffe, die einen süßen Geruchseindruck verursachen, ausgewählt sind aus der Gruppe bestehend aus:

Vanillin, Ethylvanillin, Ethylvanillinisobutyrat (= 3-Ethoxy-4-isobutyryloxybenzaldehyd), Furaneol^{®} (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd.

Bevorzugt ist der Einsatz von süß schmeckenden Stoffen ausgewählt aus der Gruppe bestehend aus
(a) Sucrose, Lactose, D-Glucose, D-Tagatose und D-Fructose, wobei diese Kohlenhydrate auch als natürliche oder künstlich hergestellte Mischung vorliegen können (z.B. als Honig, Invertzuckersirup, hochangereicherte Fructose-Sirupe aus Maisstärke [High Fructose Corn Sirup])
(b) Erythritol, Threitol, Arabitol, Ribitol, Xylitol, Sorbitol, Mannitol, Maltitol, Isomaltit, Dulcitol und Lactitol,
(c) Thaumatin, Glycin, D-Phenylalanin, D-Tryptophan,
(d) Süßstoffe aus der Gruppe Natriumcyclamat, Acesulfam K, Neohesperidindihydrochalkon, Saccharin-Natriumsalz, Aspartam, Superaspartam, Neotam, Alitam, Sucralose, Stevioside,
wobei die Menge an zugesetzter erfindungsgemäßer Aromakomposition in der Zubereitung ausreicht, den süßen Geschmackseindruck des bzw. der süß schmeckenden Stoffe, insbesondere die Anfangssüße sensorisch zu verstärken; vorzugsweise liegt die Gesamtmenge an der erfindungsgemäßen Aromakomposition dabei im Bereich von 0,1 bis 150 ppm, bevorzugt im Bereich von 1 bis 50 ppm, besonders bevorzugt im Bereich 10 bis 50 ppm bezogen auf das Gesamtgewicht der Zubereitung.

Insbesondere mit diesen Kombinationen kann (wie oben erwähnt) eine synergistische Steigerung des süßen Geschmackseindrucks erzielt werden.

Besonders bevorzugt ist eine der Ernährung, der Mundpflege oder dem Genuss dienende Zubereitung wie vorstehend definiert (insbesondere in einer der als bevorzugt gekennzeichneten Ausgestaltungen), umfassend eine Gesamtmenge im Bereich von 0,1 bis 150 ppm, bevorzugt im Bereich von 1 bis 50 ppm, besonders bevorzugt im Bereich von 10 bis 50 ppm an einer Aromakomposition wie vorstehend definiert (insbesondere in einer der als bevorzugt gekennzeichneten Ausgestaltungen), bezogen auf das Gesamtgewicht der Zubereitung.

Vorstehend wurden bevorzugte süß schmeckende Stoffe angegeben. Allgemein können süß schmeckende Stoffe (inklusive natürlicher Quellen dieser Stoffe) aber beispielsweise sein: süß schmeckende Kohlenhydrate bzw. Zucker (z.B. Sucrose (synonym zu Saccharose), Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrin) oder pflanzliche Zubereitungen enthaltend hauptsächlich diese Kohlenhydrate (z.B. aus Zuckerrübe (Beta vulgaris ssp., Zuckerfraktionen, Zuckersirup, Melasse), aus Zuckerrohr (Saccharum officinarum ssp., z.B. Melasse, Zuckersirupe), aus Zuckerahorn (Acer ssp.), aus Agave (Agavendicksaft), künstliche/enzymatische Hydrolysate von Stärke oder Sucrose (z.B. Invertzuckersirup, hochangereicherte Fructose-Sirupe aus Maisstärke), Fruchtkonzentrate (z.B. aus Birnen, Birnenkraut)), Zuckeralkohole (z.B. Glycerin, Erythritol, Threitol, Arabitol, Ribitol, Xylitol, Sorbitol, Mannitol, Maltitol, Isomaltit, Dulcitol, Lactitol), Proteine (z.B. Miraculin, Monellin, Thaumatin, Curculin, Brazzein), Süßstoffe (Magap, Natriumcyclamat, Acesulfam K, Neohesperidindihydrochalkon, Saccharin-Natriumsalz, Aspartam, Superaspartam, Neotam, Alitam, Sucralose, Stevioside, Rebaudioside, Lugduname, Carrelame, Sucrononate, Sucrooctate, Monatin, Phyllodulcin), bestimmte süßschmeckende Aminosäuren (Glycin, D-Leucin, D-Threonin, D-Asparagin, D-Phenylalanin, D-Tryptophan, L-Prolin), sonstige süß schmeckende niedermolekulare Stoffe (z.B. Hernandulcin, Isocumarine wie Phyllodulcin oder Hydrangenol, Dihydrochalkonglycoside wie Neophesperidindihydrochalkon, Glycyrrhizin, Glycyrrhetinsäureammoniumsalz oder andere Glycyrrhetinsäurederivate), Extrakte aus Süßholz (Glycyrrhizza glabra ssp.), Extrakte aus Lippia dulcis, Extrakte oder Einzelstoffe aus Momordica ssp. (insbesondere Momordica grosvenori [Luo Han Guo] und die daraus gewonnenen Mogroside), Extrakte aus *Thaumatococcus danielli* (Katemfestaude), aus Hydrangea dulcis oder aus Stevia ssp. (z.B. Stevia rebaudiana).

Die vorstehend genannten bevorzugten Aromastoffe sind Aromastoffe, die einen süßen Geruchseindruck verursachen, d. h. Aromastoffe, die zwar nicht süß im engeren Sinne schmecken, aber einen süßen Geschmack im weiteren Sinne (insbesondere inklusive Geruchswahrnehmung) suggerieren.

Bevorzugt ist eine erfindungsgemäße Zubereitung, insbesondere eine der Ernährung, der Mundpflege oder dem Genuss dienende, wobei die Gesamtmenge
- an Substanzen (i) (speichelfördernde, prickelnd, scharf und/oder warm schmeckende Alkamide) im Bereich von 0,005 bis 5 ppm, bevorzugt 0,02 bis 2 ppm, besonders bevorzugt 0,05 bis 0,5 ppm liegt,
- an Substanzen (ii) (Hesperetin wie weiter oben definiert) im Bereich von 0,5 bis 500 ppm, bevorzugt 10 bis 200 ppm, besonders bevorzugt 20 bis 100 ppm liegt
   und/oder
- an Substanzen (iii) (4-Hydroxydihydrochalkone wie weiter oben beschrieben) im Bereich von 0,5 bis 500 ppm, bevorzugt 5 bis 100 ppm, besonders bevorzugt 10 bis 40 ppm liegt,
wobei die Gesamtmenge sämtlicher Komponenten (i), (ii) und (iii) im Bereich von 0,5 bis 500 ppm, bevorzugt im Bereich 5 bis 200 ppm, besonders bevorzugt im Bereich von 10 bis 100 ppm liegt. Die angegebenen Konzentrationen beziehen sich hierbei auf die fertige, z.B. zu konsumierende Zubereitung enthaltend die erfindungsgemäße Aromakomposition.

Vorzugsweise werden erfindungsgemäße Aromakompositionen verwendet, um insbesondere die Anfangssüße eines süß schmeckenden Stoffes oder den süßen Geruchseindruck eines Aromastoffes, der einen süßen Geruchseindruck verursacht, in einer der Ernährung, der Mundpflege oder dem Genuss dienenden Zubereitung zu verstärken.

Durch die Verwendung der erfindungsgemäßen Aromakompositionen ist es insbesondere möglich, den Anteil süß schmeckender Stoffe, insbesondere aber von Zuckern wie Sucrose, Lactose, Fructose und/oder Glucose oder deren Mischungen um 5 bis 60 % (bezogen auf den bzw. die süß schmeckenden Stoffe) zu senken, verglichen mit einer Zubereitung ohne erfindungsgemäße Aromakomposition, ohne dass dabei der süße Geschmackseindruck vermindert wird.

Bevorzugt sind daher (Zucker-reduzierte) erfindungsgemäße Zubereitungen, die als süß schmeckenden Stoff bzw. süß schmeckende Stoffe der Komponente (b) einen oder mehrere Zucker umfassen, wobei die Menge an zugesetzter erfindungsgemäßer Aromakomposition ausreicht, um im Vergleich mit einer Zubereitung, die bei ansonsten identischer Zusammensetzung keine erfindungsgemäße Aromakomposition aber zumindest die 1,05-fache, vorzugsweise zumindest 1,2-fache, besonders bevorzugt zumindest 1,4-fache Menge an Zucker umfasst, den gleichen oder einen verstärkten Süßeeindruck zu vermitteln, insbesondere in der Anfangssüße. Die Zucker sind dabei vorzugsweise ausgewählt aus der Gruppe bestehend aus: Sucrose, Lactose, Glucose, Fructose und deren Mischungen.

Bevorzugte erfindungsgemäße Zubereitungen, welche zuckerfrei, zuckerreduziert oder zuckerhaltig sein können und vorzugsweise der Ernährung, der Mundpflege oder dem Genuss dienen, sind ausgewählt aus der Gruppe bestehend aus:
(A) Süßwaren, z.B. weisse, helle oder dunkle Schokoladen, gefüllte Schokoladen (beispielsweise mit aromatisierter Fondant-Masse, Typ After-Eight), Schokoladenriegel, sonstige Riegelprodukte, Kaubonbons, Fruchtgummi, Hart- und Weichkaramellen, Kaugummi, Zuckerperlen, Lutscher), Kapseln (vorzugsweise nahtlose Kapsel zum Direktverzehr, vorzugsweise mit einer Umhüllung basierend auf Gelatine und/oder Alginat), Kaugummi (z.B. in Form von Streifen, Komprimaten, Pellets, Kissen, Kugeln, Hohlkugeln),
(B) alkoholische oder nicht-alkoholische Getränke oder Instantgetränke, insbesondere Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen, Instant-Kakao-Getränke, Instant-Tee-Getränke, Instant-Kaffee-Getränke,
(C) Getreideprodukte und/oder Nussprodukte, insbesondere Frühstückscerealien, Cornflakes, Haferflocken, Schüttmüsli, Müsliriegel, Studentenfutter, süßes Popcorn, Nussriegel, Nuss-Frucht-Riegel, vorgegarte Fertigreis-Produkte,
(D) Milchprodukte, insbesondere Milchgetränke, Milcheis, Diäteis, Joghurt, Pudding, Kefir, Frischkäse, Weichkäse, Hartkäse, Trockenmilchpulver, Molke, Butter, Buttermilch, teilweise oder ganz hydrolysierte Milchprotein-haltige Produkte,
(E) Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte),
(F) Frucht- und/oder Gemüsezubereitungen, insbesondere Konfitüre, Diabetikermarmelade, Fruchteis, Fruchtsoßen, Fruchtfüllungen, Ketchup, Soßen, Trockengemüse, Tiefkühlgemüse, vorgegarte Gemüse, in Essig eingelegte Gemüse, eingekochte Gemüse,
(G) Produkte auf Fett- und Ölbasis oder Emulsionen derselben, insbesondere Mayonnaise, Remoulade, Dressings, Würzzubereitungen,
(H) Mundpflegeprodukte (Mundhygieneprodukte), insbesondere in Form von Zahnpasta, Zahncreme, Zahngel, Zahnpulver, Zahnputzflüssigkeit, Zahnputzschaum, Mundwasser, Mundwasserkonzentrat, Zahncreme und Mundwasser als 2-in-1 Produkt, Lutschbonbons, Mundspray, Zahnseide, Kaugummis oder Zahnpflegekaugummis.

Besonders relevant ist somit eine erfindungsgemäße Zubereitung, die zumindest einen süß schmeckenden Stoff, bevorzugt einen Zucker wie Sucrose, Lactose, Glucose und/oder Fructose, umfasst, wobei die Menge des süß schmeckenden Stoffs nicht ausreicht, um in einer Vergleichszubereitung, die keine erfindungsgemäße Aromakomposition umfasst, aber ansonsten identisch zusammengesetzt ist, einen befriedigenden süßen Geschmack zu vermitteln, wobei die Menge der anwesenden erfindungsgemäßen Aromakomposition in der Zubereitung ausreicht, um den süßen Geschmackseindruck des süß schmeckenden Stoffes sensorisch zu verstärken, vorzugsweise soweit, dass eine befriedigende Anfangssüße vermittelt wird.

Bevorzugte erfindungsgemäße Zubereitungen sind der Ernährung, der Mundpflege oder dem Genuss dienende Zubereitungen, hinsichtlich deren Zusammensetzungen das Vorgesagte gilt.

Die erfindungsgemäßen der Ernährung, der Mundpflege oder dem Genuss dienenden Zubereitungen sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel) oder wieder aus der Mundhöhle entfernt zu werden (z.B. Kaugummis oder Zahnpasta). Es versteht sich, dass der Einsatz der erfindungsgemäßen Aromakompositionen für jede Art solcher Produkte vorgesehen ist. Zu diesen Produkten gehören dabei sämtliche Stoffe oder Erzeugnisse, die dazu bestimmt sind, in verarbeitetem, teilweise verarbeitetem oder unverarbeitetem Zustand vom Menschen in die Mundhöhle aufgenommen zu werden. Hierzu zählen auch Stoffe, die Lebensmitteln bei ihrer Herstellung, Verarbeitung oder Bearbeitung zugesetzt werden und dazu vorgesehen sind, in die menschliche Mundhöhle eingebracht zu werden.

Es versteht sich, dass die erfindungsgemäßen Aromakompositionen insbesondere in Lebensmitteln eingesetzt werden können. Im Rahmen des vorliegenden Textes werden unter einem "Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Insbesondere werden die erfindungsgemäßen Aromakompositionen in verzehrfertigen Lebensmitteln eingesetzt. Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch entsprechende Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

Die erfindungsgemäßen Aromakompositionen können auch in Lebensmittel-Halbfertigwaren eingesetzt werden. Der Begriff Lebensmittel-Halbfertigware bezieht sich hierbei auf Lebensmittel, die dazu bestimmt sind, erst im weiter verarbeiteteten Zustand, nach Hinzufügen von für den sensorischen Eindruck (mit)entscheidenden Aroma- oder Geschmacksstoffen verzehrt zu werden.

Unter einer der Mundpflege dienenden Zubereitung (Mundpflegeprodukt, auch Mundhygieneprodukt oder mundhygienische Zubereitung genannt) im Sinne der Erfindung wird eine Zubereitung zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind Cremes, Gele, Pasten, Schäume, Emulsionen, Suspensionen, Areosole, Sprays als auch Kapseln, Granulate, Pastillen, Tabletten, Bonbons oder Kaugummis, ohne dass diese Aufzählung für die Zwecke dieser Erfindung limitierend verstanden werden soll.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Die vorliegende Erfindung betrifft insbesondere eine der Ernährung, der Mundpflege oder dem Genuss dienende Zubereitung umfassend
eine Komponente (a), die aus der erfindungsgemäßen Aromakomposition besteht
eine Komponente (b) (d. h. eine oder mehrere süß schmeckende Stoffe), umfassend oder bestehend aus einem oder mehreren Zuckern,
sowie gegebenenfalls
Komponente (c) (d. h. einen oder mehrere Aromastoffe, die einen süßen Geruchseindruck verursachen)
wobei die Gesamtmenge an Komponente (a) in der Zubereitung
ausreicht, um im Vergleich mit einer Zubereitung die bei ansonsten identischer Zusammensetzung keine der erfindungsgemäßen Aromakompositionen aber zumindest die 1,05-fache Menge an Zucker umfasst, den gleichen oder einen verstärkten Süßeeindruck zu vermitteln
und/oder
im Bereich von 0,5 bis 500 ppm liegt.

Weitere bevorzugte erfindungsgemäße Zubereitungen sind Halbfertigwaren, Riech-, andere Aroma- oder Geschmacksstoffkompositionen oder Würzmischungen.

Der Begriff Halbfertigwaren umfasst hierbei insbesondere Lebensmittel-Halbfertigwaren, wie vorstehend definiert. Erfindungsgemäße Halbfertigwaren liegen vorzugsweise in sprühgetrockneter Form vor. Erfindungsgemäße Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Erfindungsgemäße Zubereitungen, die als Halbfertigwaren vorliegen, können insbesondere zur Verstärkung des süßen Geschmackseindrucks von der Ernährung, der Mundpflege oder dem Genuss dienenden Fertigware-Zubereitungen dienen, die unter Verwendung der Halbfertigware-Zubereitung hergestellt werden.

Erfindungsgemäße sprühgetrocknete feste Zubereitungen als Halbfertigwaren sind besonders gut zur Herstellung von erfindungsgemäßen Zubereitungen geeignet, die insbesondere der Ernährung der Mundpflege oder dem Genuss dienen können. In den sprühgetrockneten Halbfertigwaren wird nämlich die Löslichkeit der erfindungsgemäßen Aromakompositionen durch die Träger- und/oder Hilfsstoffe, insbesondere durch Maltodextrin, Stärke, natürliche oder künstliche Polysaccharide und/oder Pflanzengummen wie modifizierte Stärken oder Gummi Arabicum, wesentlich verbessert. In den erfindungsgemäßen sprühgetrockneten festen Halbfertigwaren sind vorzugsweise 50 bis 95 Gew.-% Trägerstoffe, insbesondere Maltodextrin und/oder Stärke, 5 bis 40 Gew.-% Hilfsstoffe, bevorzugt natürliche oder künstliche Polysaccharide und/oder Pflanzengummen wie modifizierte Stärken oder Gummi Arabicum und 1 bis 45 Gew.-% an erfindungsgemäßen Aromakompositionen enthalten, bezogen auf die Gesamtmenge der sprühgetrockneten festen Zubereitung.

Erfindungsgemäße Zubereitungen, die aus der Gruppe bestehend aus Halbfertigwaren, Riech-, Aroma- oder Geschmacksstoffkompositionen oder Würzmischungen ausgewählt sind, umfassen vorzugsweise eine Gesamtmenge im Bereich von 0,0001 Gew.-% bis 95 Gew.-%, bevorzugt 0,001 Gew.-% bis 80 Gew.-%, besonders bevorzugt 0,001 Gew.-% bis 50 Gew.-%, an erfindungsgemäßen Aromakompositionen, bezogen auf das Gesamtgewicht der Zubereitung.

Bevorzugt können die erfindungsgemäßen Zubereitungen auch weitere Aromakompositionen enthalten, um den Geschmack und/oder Geruch der Zubereitung abzurunden und zu verfeinern. Geeignete weitere Aromakompositionen enthalten z.B. synthetische, natürliche oder naturidentische Aroma-, Riech- und Geschmacksstoffe sowie geeignete Hilfs- und Trägerstoffe. Besonders bevorzugt sind erfindungsgemäße Zubereitungen, die einen oder mehrere Aromastoffe, die einen "süßen" Geruchseindruck verursachen, enthalten (siehe dazu oben). Bevorzugte weitere synthetische, natürliche oder naturidentische Aroma-, Riech- und Geschmacksstoffe sind weitere speichelfördernde, prickelnd, scharf und/oder warm schmeckende Substanzen wie beispielsweise Genusssäuren (beispielsweise Zitronensäure, Äpfelsäure, Weinsäure), Capsaicin, Dihydrocapsaicin, Gingerole, Paradole, Shogaole, Piperin, Carbonsäure-N-vanillylamide, insbesondere Nonansäure-N-vanillylamid, 2-Alkensäureamide, insbesondere 2-Nonensäure-N-isobutylamid, 2-Nonensäure-N-4-hydroxy-3-methoxy-phenylamid, Alkylether von 4-Hydroxy-3-methoxybenzylalkohol, insbesonders 4-Hydroxy-3-methoxybenzyl-n-butylether, Alkylether von 4-Acyloxy-3-methoxy-benzylalkohol, insbesondere 4-Acetyloxy-3-methoxybenzyl-n-butylether und 4-Acetyl-oxy-3-methoxybenzyl-n-hexylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, (4-Hydroxy-3-methoxyphenyl)essigsäureamide, insbesondere (4-Hydroxy-3-methoxyphenyl)essigsäure-N-n-octylamid, Ferulasäurephenethylamiden, insbesondere N-Feruloyl-3-methoxytyramin oder Rubemamin, Nicotinaldehyd, Methylnicotinat, Propylnicotinat, 2-Butoxyethylnicotinat, Benzylnicotinat, 1-Acetoxychavicol, Polygodial, 1-sodrimeninol und Pilocarpin.

Erfindungsgemäße Halbfertigwaren umfassen regelmäßig weitere Geschmacks- und/oder Aromastoffe, insbesondere Aromastoffe die einen süßen Geruchseindruck verursachen (vergleiche weiter oben), sowie geeignete Lösungsmittel (z.B. Ethanol, Glycerin, 1,2-Propylenglycol, Alkylester von Milchsäure, Ethylester von organischen Fruchtsäuren wie Diethylmalonat, Diethyltartrat, Diethylmalat, Triethylcitrat, Diethylsuccinat, Diethylfumarat, Diethylmaleat) und weitere Hilfsstoffe (z.B. Farbstoffe, Pigmente, Antioxidantien, Konservierungsmittel, Emulgatoren, Viskositäts-beeinflussende Stoffe).

Erfindungsgemäße sprühgetrocknete feste Halbfertigwaren umfassen vorzugsweise 1 bis 50 Gew.-% erfindungsgemäße Aromakomposition, bezogen auf das Gesamtgewicht der Zubereitung, 0 bis 10 Gew.-%, vorzugsweise 1 bis 10 Gew.-% sonstige Aromen, 50 bis 99 Gew.-% Trägerstoffe und 0 bis 50 Gew.-% vorzugsweise 1 bis 50 Gew.-% weitere Hilfsmittel und/oder Stabilisatoren, jeweils bezogen auf das Gesamtgewicht der Zubereitung.

Vorteilhafte Trägerstoffe in den erfindungsgemäßen sprühgetrockneten festen Zubereitungen sind Kohlenhydrate und/oder Kohlenhydratpolymere (Polysaccharide). Als bevorzugte Trägerstoffe in den erfindungsgemäß einzusetzenden Aromapartikeln sind beispielsweise Hydrokolloide wie Stärken, abgebaute Stärken, chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum, Dextrine und Maltodextrine zu nennen.

Der Zersetzungsgrad der Stärke wird mit der Kennzahl "Dextrose-Equivalent" (DE) gemessen, welche die Grenzwerte 0 für das langkettige Glucosepolymer Stärke und 100 für die reine Glucose annehmen kann.

Besonders bevorzugte Trägerstoffe für die erfindungsgemäßen sprühgetrockneten festen Zubereitungen sind Maltodextrine, wobei Maltodextrine mit DE-Werten im Bereich 10 bis 30 hier wiederum vorteilhaft sind.

Es wurde bereits erwähnt, dass sprühgetrocknete feste Halbfertigwaren besonders gut zur Herstellung erfindungsgemäßer Zubereitungen geeignet sind, welche der Ernährung, der Mundpflege oder dem Genuss dienen sollen.

Eine bevorzugte erfindungsgemäße Zubereitung umfasst als zusätzliche Komponente (d)
einen oder mehrere Ester ausgewählt aus der Gruppe bestehend aus Milchsäure-C₁-C₆-Estern, Weinsäure-di-C₁-C₄-Estern, Bersteinsäure-di-C₁-C₄-Estern, Malonsäure-di-C₁-C₄-Estern, Äpfelsäure-di-C₁-C₄-Estern, Citronensäure-di-C₁-C₄-Estern und Citronensäure-tri-C₁-C₄-Estern,
und/oder
ein oder mehrere Lösungsmittel ausgewählt aus der Gruppe bestehend aus 1,2-Propylenglycol, Dimethylsulfoxid, Ethanol, und Ethanol/Wasser-Mischungen.

Neben der zusätzlichen Komponente (d) sind vorzugsweise ein oder mehrere weitere Aromastoffe vorhanden, insbesondere Aromastoffe, die einen süßen Geruchseindruck verursachen und dabei vorzugsweise aus der oben angegebenen Gruppe solcher Aromastoffe ausgewählt sind.

Besonders bevorzugt für den Einsatz in den erfindungsgemäßen Aromakompositionen sind Ester ausgewählt aus der Gruppe bestehend aus Ethyllactat, n-Propyllactat, n-Butyllactat, Diethyltartrat, Dimethylsuccinat, Diethylsuccinat, Dimethylmalonat, Diethylmalonat, Dimethylmalat, Diethylmalat, und Triethylcitrat sowie das Lösungsmittel 1,2-Propylenglycol.

Die Anwesenheit der vorstehend genannten Ester bzw. Lösungsmittel in erfindungsgemäßen Aromakompositionen bewirkt eine sehr gute Löslichkeit und verhindert eine nennenswerte Neigung zur Rekristallisation dererfindungsgemäßen Aromakompositionen. Solche erfindungsgemäßen Aromakompositionen sind daher besonders geeignet zur Einarbeitung in erfindungsgemäße, der Ernährung, der Mundpflege oder dem Genuss dienende Zubereitungen. Hinsichtlich der bevorzugten Konzentrationen der erfindungsgemäßen Aromakompositionen sei auf die obigen Ausführungen verwiesen.

Erfindungsgemäße Zubereitungen, welche der Ernährung, der Mundpflege oder dem Genuss dienen, werden vorzugsweise hergestellt, indem die einzelnen Substanzen (i), (ii) und/oder (iii) der erfindungsgemäßen Aromakompositionen als reine Stoffe , als Lösung (z.B. in Ethanol, Wasser, 1,2-Propylenglycol, Dimethylsulfoxid, ggf. in Anwesenheit eines der oben angegebenen Ester oder Lösungsmittel) oder in Form eines Gemisches mit einem festen oder flüssigen Trägerstoff (z.B. Maltodextrin, Stärke, Silicagel), weiteren Aromen oder Aromastoffen und gegebenenfalls weiteren Hilfsmitteln und/oder Stabilisatoren (z.B. natürliche oder künstliche Polysaccharide und/oder Pflanzengummen wie modifizierte Stärken oder Gummi Arabicum), d.h. in Form einer Halbfertigware, in eine der Ernährung, der Mundpflege oder dem Genuss dienende Basis-Zubereitung eingearbeitet werden. Vorteilhafterweise kann eine als Lösung und/oder Suspension oder Emulsion vorliegende erfindungsgemäße Zubereitung zunächst durch Sprühtrocknung in eine feste erfindungsgemäße Zubereitung (Halbfertigware) überführt werden, bevor diese dann wiederum zur Herstellung einer erfindungsgemäßen, der Ernährung, der Mundpflege oder dem Genuss dienenden Zubereitung eingesetzt wird. Hinsichtlich der besonderen Eignung sprühgetrockneter Halbfertigwaren zur Herstellung von der Ernährung, der Mundpflege oder dem Genuss dienenden Zubereitungen sei auf die obigen Ausführungen verwiesen.

Gemäß einer weiteren bevorzugten Ausführungsform werden zur Herstellung erfindungsgemäßer Zubereitungen die erfindungsgemäßen Aromakompositionen und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulate oder Extrudate aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) eingearbeitet. Dabei können je nach Matrix die Produkte durch Sprühtrocknung, Sprühgranulation, Schmelzgranulation, Wirbelschichtverfahren (z.B. gemäß WO 97/16078 oder WO 2004/022642), Wirbelschichtsprühgranulation (z.B. gemäß WO 00/36931 oder US 4,946,654), Koazervation, Koagulation, Extrusion, Schmelzextrusion (z.B. gemäß WO 2003/092412, EP 1 123 660 oder EP 1 034 705), Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung werden die erfindungsgemäßen Aromakompositionen zunächst mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

In manchen Fällen bevorzugt ist eine erfindungsgemäße Zubereitung, bei der die Matrix so gewählt ist, dass die erfindungsgemäße Aromakomposition verzögert von der Matrix freigegeben werden, so dass eine langanhaltende Wirkung erzielt wird. Besonders bevorzugt ist insoweit eine Fett-, Wachs-, Polysaccharid- oder Proteinmatrix.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Amylose, Amylopektin, Inulin, Xylane, Cellulose), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B.

Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, Kühlwirkstoffe wie z.B. Menthol, Mentholderivate (z.B. L-Menthol, L-Menthyllactat, L-Menthylglutarat, L-Menthylsuccinat) oder Cubebol, synthetische, natürliche oder naturidentische Aromastoffe oder Riechstoffe sowie Geruchskorrigentien.

Zahnpflegemittel (als Beispiel für ein erfindungsgemäßes Mundpflegeprodukt), die eine erfindungsgemäße Aromakomposition enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemittel wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffe wie z.B. Menthol, Mentholderivate (z.B. L-Menthol, L-Menthyllactat, L-Menthylalkylcarbonate, Menthonketale, Menthancarbonsäureamide, L-Menthylglutarat, L-Menthylsuccinat), 2,2,2-Trialkylessigsäureamide (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartäre Ammoniumfluoride, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), welche die erfindungsgemäßen Aromakompositionen enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffe wie z.B. Menthol, Mentholderivate (z.B. L-Menthol, L-Menthyllactat, L-Menthylalkylcarbonate, Menthonketale, Menthancarbonsäureamide, L-Menthylglutarat, L-Menthylsuccinat), 2,2,2-Trialkylessigsäureamide (z.B. 2,2-Diisopropylpropionsäuremethylamid), Icilin und Icilin-Derivate, Coffein, Theobromin, Theophyllin, Feuchthaltemittel, Verdicker, Emulgatoren, Aromen und Stabilisatoren oder Geruchskorrigentien.

Eine besonders bevorzugte erfindungsgemäße Zubereitung umfasst zumindest eine weitere Substanz zum Maskieren oder Vermindern eines bitteren, metallischen, kalkigen, sauren oder adstringierenden Geschmackseindrucks oder zum Verstärken eines süßen, salzigen oder Umami-Geschmackseindrucks. Es werden somit die erfindungsgemäßen Aromakompositionen in Kombination mit zumindest einer (weiteren) zur Maskierung oder Verminderung eines unangenehmen (bitteren, metallischen, kalkigen, sauren, adstringierenden) Geschmackseindrucks oder zur Verstärkung eines angenehmen Geschmackseindrucks (süß, salzig, Umami) geeigneten Substanz verwendet. Diese speziellen Zubereitungen sind hervorragend geeignet, eine besonders wirksame Verstärkung der Anfangssüße in den erfindungsgemäßen Zubereitungen enthaltend süß schmeckende Stoffe zu erreichen. Insbesondere die Kombination der erfindungsgemäßen Aromakompositionen mit Geschmackskorrigenzen für unangenehme, insbesondere bittere Geschmackseindrücke, oder Geschmacksverstärkern für angenehme, insbesondere süße Geschmackseindrücke, ist bevorzugt.

Die (weiteren) Geschmackskorrigenzien werden z. B. aus der folgenden Liste ausgewählt: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine gemäß WO2006/106023 sowie den darauf basierenden Dokumenten (Symrise) (z.B. 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen gemäß US Provisional Application 60/728,744 sowie den darauf basierenden Dokumenten (Symrise) (PCT/EP2006/067120), Diacetyltrimere gemäß PCT/EP 2005/056355 sowie den darauf basierenden Dokumenten (Symrise), Divanillin, Gemische von Molkeproteinen mit Lecithinen und/oder bittermaskierende Substanzen wie Gingerdione gemäß WO2007/003527.

Es wurde bereits mehrfach ausgeführt, dass erfindungsgemäße Zubereitungen insbesondere aus der Gruppe ausgewählt sind, die aus der Ernährung, der Mundpflege oder dem Genuss dienenden Zubereitungen, Halbfertigwaren, Riech- Aroma- oder Geschmackstoffskompositionen oder Würzmischungen besteht. Nachfolgend werden bevorzugte erfindungsgemäße Zubereitungen angegeben: Backwaren (z.B. Brot, Trockenkekse, Kuchen, Muffins, Waffeln, Backmischungen, sonstiges Gebäck), Süßwaren (z.B. weisse, helle oder dunkle Schokoladen, gefüllte Schokoladen (beispielsweise mit aromatisierter Fondant-Masse, Typ After-Eight), Schokoladenriegel, sonstige Riegelprodukte, Kaubonbons, Fruchtgummi, Hart- und Weichkaramellen, Kaugummi, Zuckerperlen, Lutscher), Kapseln (vorzugsweise nahtlose Kapsel zum Direktverzehr, vorzugsweise mit einer Umhüllung basierend auf Gelatine und/oder Alginat), Fettmassen (z.B. Backwarenfüllungen für z.B. Keksfüllungen, Schokoladenfettfüllungen, Riegelfettfüllungen), Aufstreumischungen (Toppings), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke oder Instantpulver (z.B. Instant-Kakao-Getränke, Instant-Tee-Getränke, Instant-Kaffeegetränke, Instant Desserts in Pulverform wie Puddingspulver oder Götterspeise), Fleischprodukte (z.B. Schinken, Frischwurst- oder Rohwurstzubereitungen, gewürzte oder marinierte Frisch- oder Pökelfleischprodukte), Eier oder Eiprodukte (z.B. Trockeneipulver), Getreideprodukte und/oder Nussprodukte (z.B. Frühstückscerealien, Cornflakes, Haferflocken, Schüttmüsli, Müsliriegel, Studentenfutter, süßes Popcorn, Nussriegel, Nuss-Frucht-Riegel, vorgegarte Fertigreis-Produkte), Milchprodukte (z.B. Milchgetränke, Milcheis, Joghurt, Pudding, Kefir, Frischkäse, Weichkäse, Hartkäse, Trockenmilchpulver, Molke, Butter, Buttermilch, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder entsprechende Emulsionen (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Die vorliegende Erfindung betrifft auch ein Verfahren zum Verstärken des süßen Geschmacks bzw. der Anfangssüße eines süß schmeckenden Stoffes oder des süßen Geruchseindruckes bzw. der Anfangssüße eines Aromastoffes, der einen süßen Geruchseindruck verursacht, mit folgendem Schritt:
- Mischen eines oder mehrerer süß schmeckender Stoffe (Komponente (b), wie oben definiert) oder eines oder mehrerer Aromastoffe, der beziehungsweise die einen süßen Geruchseindruck verursachen (Komponente (c), wie oben definiert), mit einer Gesamtmenge einer erfindungsgemäßen Aromakomposition (Komponente (a) wie oben definiert), wobei die Gesamtmenge an erfindungsgemäßer Aromakomposition (Komponente (a)) in der Zubereitung ausreicht, den süßen Geschmackseindruck des bzw. der süß schmeckenden Stoffe (b) bzw. den süßen Geruchseindruck des bzw. der Aromastoffe (c), die einen süßen Geruchseindruck verursachen, und insbesondere die Anfangssüße sensorisch zu verstärken, vorzugsweise überproportional

Vorzugsweise werden hierbei eine, zwei oder insbesondere alle der Komponenten (a), (b) und (c) in einer ihrer bevorzugten Ausgestaltungen wie vorstehend definiert eingesetzt.

Zu den bevorzugten Mengen an erfindungsgemäßen Aromakompositionen siehe oben. Eine Gesamtkonzentration von zumindest 10 ppm und höchstens 100 ppm in einer gebrauchsfertigen, der Mundpflege dienenden Zubereitung bzw. einer verzehrfertigen, der Ernährung oder dem Genuss dienenden Zubereitung ist dabei oftmals besonders bevorzugt.

Insgesamt gelten jedoch auch für das erfindungsgemäße Verfahren die vorstehend hinsichtlich der erfindungsgemäßen Aromakomposition, der erfindungsgemäßen Verwendung und der erfindungsgemäßen Zubereitung gemachten Ausführungen.

Weiterhin betrifft die vorliegende Erfindung die Verwendung von einem oder mehreren Alkamiden (i) ausgewählt aus der Gruppe bestehend aus:

2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,4E-Decadiensäure-N-([2S]-2-methylbutyl)amid, 2E,4E-Decadiensäure-N-([2S]-2-methylbutyl)amid, 2E,4E-Decadiensäure-N-([2R]-2-methylbutylamid), 2E,4Z-Decadiensäure-N-(2-methylbutyl)amid, 2E,4E-Decadiensäure-N-piperid (Achilleamid), 2E,4E-Decadiensäure-N-piperid (Sarmentin), 2E-Decensäure-N-isobutylamid, 3E-Decensäure-N-isobutylamid, 3E-Nonensäure-N-isobutylamid, 2E,6Z,8E-Decatriensäure-N-isobutylamid (Spilanthol), 2E,6Z,8E-Decatriensäure-N-([2S]-2-methylbutyl)amid (Homospilanthol), 2E,6Z,8E-Decatriensäure-N-([2R]-2-methylbutyl)amid, 2E-Decen-4-insäure-N-isobutylamid, 2Z-Decen-4-insäure-N-isobutylamid, 2E,6Z,8E,10E-Dodecatetraensäure-N-(2-methylpropyl)amid (α-Sanshool), 2E,6Z,8E,10E-Dodecatetraensäure-N-(2-hydroxy-2-methylpropyl)amid (α-Hydroxysanshool), 2E,6E,8E,10E-Dodecatetraensäure-N-(2-hydroxy-2-methylpropyl)amid (β-Hydroxysanshool), 2E,4E,8Z,10E,12E-Tetradecapentaensäure-N-(2-hydroxy-2-methylpropyl)amid (γ-Hydroxysanshool), 2E,4E,8E,10E,12E-Tetradecapentaensäure-N-(2-hydroxy-2-methylpropyl)amid (γ-Hydroxyisosanshool), 2E,4E,8Z,10E,12E-Tetradecapentaensäure-N-(2-methyl-2-propenyl)amid (γ-dehydrosanshool), 2E,4E,8Z,10E,12E-Tetradecapentaensäure-N-(2-methylpropyl)amid (γ-Sanshool), 2E,4E,8Z,11Z-Tetradecatetraensäure-N-(2-hydroxy-2-methylpropyl)amid (Bungeanool), 2E,4E,8Z,11 E-Tetradecatetraensäure-N-(2-hydroxy-2-methylpropyl)amid (Isobungeanool), 2E,4E,8Z-Tetradecatriensäure-N-(2-hydroxy-2-methylpropyl)amid (Dihydrobungeanool) und 2E,4E -Tetradecadiensäure-N-(2-hydroxy-2-methylpropyl)amid (Tetrahydrobungeanool) zur Verstärkung der Anfangssüße einer Zubereitung umfassend
(ii) Hesperetin der Formel (I) 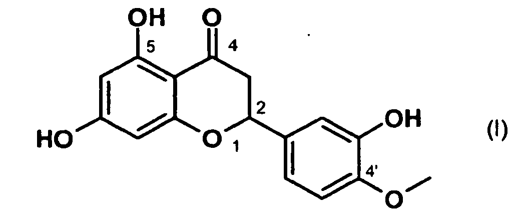 wobei das Hesperetin der Formel (I) als (2*S*)-Enantiomer, (2*R*)-Enantiomer oder beliebige Mischung der beiden Enantiomere vorliegt, und/oder dessen Salzen
   und/oder
(iii)
   - ein 4-Hydroxydihydrochalkon der Formel (II) 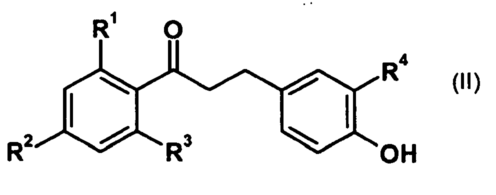 wobei
      R¹, R², R³ und R⁴ unabhängig voneinander jeweils H, OH oder O-Alkyl bedeuten, mit der Maßgabe, dass mindestens einer der Reste R¹, R² oder R³ OH bedeutet,
   - ein Salz eines solchen 4-Hydroxydihydrochalkons der Formel (II),
   - eine Mischung umfassend oder bestehend aus zwei oder mehr unterschiedlichen 4-Hydroxydihydrochalkonen der Formel (II), wobei R¹, R², R³ und R⁴ jeweils die oben angegebene Bedeutung haben,
   - eine Mischung umfassend oder bestehend aus Salzen von zwei oder mehr unterschiedlichen 4-Hydroxydihydrochalkonen der Formel (II), wobei R¹, R², R³ und R⁴ jeweils die oben angegebene Bedeutung haben
      oder
   - eine Mischung umfassend oder bestehend aus einem 4-Hydroxydihydrochalkon der Formel (II) oder zwei oder mehr unterschiedlichen 4-Hydroxydihydrochalkonen der Formel (II), wobei R¹, R², R³ und R⁴ jeweils die oben angegebene Bedeutung haben, und
einem Salz eines 4-Hydroxydihydrochalkons der Formel (II) oder zwei oder mehr Salzen von unterschiedlichen 4-Hydroxydihydrochalkonen der Formel (II), wobei R¹, R², R³ und R⁴ jeweils die oben angegebene Bedeutung haben,
sowie umfassend
(b) einen oder mehrere süß schmeckende Stoffe
   und/oder
(c) einen oder mehrere Aromastoffe, die einen süßen Geruchseindruck verursachen.

### Beispiele

Die Beispiele dienen zur Verdeutlichung der Erfindung, ohne diese damit einzuschränken. Sofern nicht anders angegeben, beziehen sich alle Angaben auf das Gewicht.

### Anwendungsbeispiel 1: Aromakompositionen

| | **Zubereitung (Einsatz in Gew.-%)** | | | | |
|---|---|---|---|---|---|
| **Inhaltsstoff** | **A** | **B** | **C** | **D** | **E** |
| 10 Gew.-% Pellitorin (z.B. nach WO 2004/043960) in 1,2-Propylenglycol/Diethylmalonat (Inhaltsstoff (i)) | 0,25 % | | 0,25 % | 0,25 % | 0,5 % |
| Jambu-Oleoresin, enthaltend 30 Gew.-% Spilanthol in 1,2-Propylenglycol (Robertet) (Inhaltsstoff (1)) | | 0,1 % | 0,1 % | | |
| Hesperetin (Inhaltsstoff (ii)) | 2,5 % | 2,5 % | 2,5 % | 2,5 % | 5 % |
| Phloretin (Verbindung 4) (Inhaltsstoff (iii)) | - | 2,5 % | 2,5 % | 2,5 % | - |
| Propylenglycol | ad 100 | ad 100 | ad 100 | ad 100 | - |
| Ethanol | - | - | - | - | ad 100 |

Die Inhaltsstoffe (i), (ii) und (iii) (Stoffe bzw. Lösungen) werden in den oben angegebenen Mengenverhältnissen gemischt und dann mit Propylenglycol oder Ethanol aufgenommen und durch leichtes Erwärmen vollständig gelöst.

### Anwendungsbeispiel 2: Sprühgetrocknete Zubereitungen als Halbfertigwaren zur Aromatisierung von Fertigwaren

| | **Zubereitung (Einsatz in Gew.-%)** | | | | |
|---|---|---|---|---|---|
| **Inhaltsstoff** | **A** | **B** | **C** | **D** | **E** |
| Trinkwasser | 60,72 % | 60,72 % | 62,94% | 65,14% | 60,76 % |
| Maltodextrin aus Weizen | 24,3% | 24,3% | 24,3% | 24,3% | 24,3% |
| Gummi Arabicum | 6,1 % | 6,1 % | 6,1 % | 6,1 % | 6,1 % |
| Pellitorin nach WO 2004/043960 (Inhaltsstoff (i)) | 0,08 % | 0,08 % | 0,06 % | 0,06 % | 0,04 % |
| Hesperetin (Inhaltsstoff (ii)) | - | 4,4 % | 4,4 % | 2,2 % | 8,8 % |
| Phloretin (Verbindung 4) (Inhaltsstoff (iii)) | 8,8 % | 4,4 % | 2,2 % | 2,2 % | - |

Das Trinkwasser wird in einem Behälter vorgelegt und das Maltodextrin und das Gummi Arabicum darin gelöst. Anschließend werden die Inhaltsstoffe (i), (ii) und (iii)mit einem Mixer (Turrax) in die oben beschriebene Trägerstofflösung emulgiert. Die Temperatur des resultierenden Gemisches sollte 30°C nicht überschreiten. Das Gemisch wird dann sprühgetrocknet (Solltemperatur Eingang: 185 - 195°C, Solltemperatur Ausgang: 70 - 75°C). Die sprühgetrocknete Halbfertigware enthält ca. 18 - 22 % der erfindungsgemäßen Aromakompositionen.

Analog können auch sprühgetrocknete Zubereitungen mit erfindungsgemäßen anderen Aromakompositionen hergestellt werden.

### Anwendungsbeispiel 3: Kombinationen mit süß schmeckenden Stoffen als Süßungsmittel

| | **Zubereitung (Einsatz in Gew.-%)** | | | | | | | |
|---|---|---|---|---|---|---|---|---|
| **Inhaltsstoff** | **A** | **B** | **C** | **D** | **E** | **F** | **G** | **H** |
| Sucrose | 88 | 89 | 89 | 89 | 89 | | | 50 |
| Fructose | 10 | 10 | 10 | | | | | |
| Tagatose | | | | 10 | 10 | | | |
| High Fructose Corn Sirup | | | | | | 99 | | |
| Maltitol | | | | | | | 99 | |
| Sorbitol | | | | | | | | 49 |
| Aromakomposition A nach Anwendungsbeispiel 1 | 2 | | | | | 1 | | |
| Aromakomposition B nach Anwendungsbeispiel 1 | | 1 | | | | | | |
| Aromakomposition C nach Anwendungsbeispiel 1 | | | 1 | | | | | |
| Aromakomposition D nach Anwendungsbeispiel 1 | | | | 1 | | | | 1 |
| Aromakomposition E nach Anwendungsbeispiel 1 | | | | | 1 | | 1 | |

Die Inhaltsstoffe werden in der angegebenen Reihenfolge gemischt; bevorzugt werden die Aromakompositionen durch Aufsprühen eingebracht. Das resultierende Produkt kann als Süßungsmittel für Nahrungs- oder Genussmittel, z.B. Kaffee oder Tee eingesetzt werden.

Als Beispiel für die Anwendung wird Tee und das Produkt gemischt und in Teebeutel aus Filterpapier abgepackt. Zur Anwendung einen Teebeutel mit 100 - 250 mL kochendem Wasser aufgießen und 2 - 5 min ziehen lassen.

### Anwendungsbeispiel 4: Aromamischungen zur Verstärkung der Süße

| | **Zubereitung (Einsatz in Gew.-%)** | | | | | | | | | |
|---|---|---|---|---|---|---|---|---|---|---|
| **Inhaltsstoff** | **A** | **B** | **C** | **D** | **E** | **F** | **G** | **H** | **I** | **J** |
| Vanillearoma (z.B. erhältlich von Symrise) | 75,00 | | 75,00 | | | | | | | |
| Zuckeraroma Typ Black Treacle | | 2,00 | | | | | | | | |
| Ethyllactat | | | | 1,00 | 0,50 | 0,050 | | 0,50 | 1,00 | 0,05 |
| n-Propyllactat | | | | 0,50 | | | | 0,50 | 0,50 | |
| n-Butyllactat | | | | 0,30 | 0,30 | 0,030 | 1,80 | 0,30 | 0,30 | 0,03 |
| Diethylmalat | | | | 1,00 | | | 1,00 | 0,50 | 1,00 | |
| Diethyltartrat | | | | 0,50 | | | | 0,50 | 0,50 | |
| Diethylsuccinat | | | | 0,50 | | | | | 0,50 | 10,0 |
| Diethylmalonat | | | | 0,50 | | | 2,00 | | 0,50 | |
| Triethylcitrat | | | | 0,50 | | | | 0,50 | 0,50 | |
| Milchsäure | | | | 1,00 | 2,00 | 0,20 | 2,00 | | 2,00 | 0,20 |
| 10 Gew.-% Pellitorin (z.B. nach WO 2004043960) in 1,2-Propylenglycol/Diethylmalonat (Inhaltsstoff (i)) | 0,05 | 0,10 | 0,03 | 0,25 | 0,30 | 0,20 | 0,10 | 0,50 | 0,10 | 0,30 |
| Hesperetin (Inhaltsstoff (ii)) | | 2,45 | 0,30 | | 1,25 | | 1,25 | | 1,50 | 2,50 |
| Phloretin (Verbindung 4) (Inhaltsstoff (iii)) | 0,625 | | 0,325 | 2,50 | 1,25 | 2,50 | 1,25 | 5,00 | 1,00 | 2,50 |
| 1,2-Propylenglycol | ad 100 | ad 100 | ad 100 | ad 100 | ad 100 | ad 100 | ad 100 | ad 100 | ad 100 | ad 100 |

Die in der Tabelle angegebenen Komponenten werden in der angegebenen Reihenfolge durch Rühren gemischt und ggfs. durch Erwärmung auf 20-50 °C vollständig homogenisiert. Man erhält klare, meist farblose oder gelbliche Lösungen, die als Aroma eingesetzt werden können.

### Anwendungsbeispiel 5: Sojamilchgetränke

Vergleichszubereitung mit Zucker (A)

Vergleichszubereitung mit reduziertem Zuckeranteil und Hesperetin (B)

Erfindungsgemäße Zubereitungen mit reduziertem Zuckeranteil und erfindungsgemäßen Aromakompositionen (C-E)

| | **Zubereitung (Angaben als Gew.-%)** | | | | |
|---|---|---|---|---|---|
| **Inhaltsstoff** | A | B | C | D | E |
| Sucrose | 6 % | 4,8 % | 4,8 % | 4,8 % | 4,8 % |
| Hesperetin | - | 50 ppm | - | - | - |
| Aromakomposition A nach Anwendungsbeispiel 1 | - | - | 0,2 % | - | 0,2 % |
| Aromakomposition D nach Anwendungsbeispiel 1 | - | - | - | 0,2 % | - |
| Sahne-Aroma (z.B. erhältlich von Symrise) | - | - | - | - | 0,002% |
| Sojamilch, Marke Sojasun, nichtaromatisiert und nicht gesüßt | zu 100 % auffüllen | zu 100 % auffüllen | zu 100 % auffüllen | zu 100 % auffüllen | zu 100 % auffüllen |

Verglichen mit Zubereitung A war in Zubereitung B die Süße ähnlich, aber die Anfangssüße zu gering. In Zubereitung C, D und E war die Gesamtsüße und die Anfangsüße ähnlich zu Zubereitung A.

### Anwendungsbeispiel 6: Kaugummis

### Anwendungsbeispiel 6a:

| **Teil** | **Inhaltsstoff** | **Einsatz in Gew.%** |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70%ige wässrige Lösung | 14,00 |
| | Glycerin | 1,00 |
| D | Krauseminzaroma | 0,80 |
| | Aromakomposition A aus Anwendungsbeispiel 1 | 0,20 |

Teile A bis D werden gemischt und intensiv geknetet. Die Rohmasse kann z.B. in Form von dünnen Streifen zu verzehrsfertigen Kaugummis verarbeitet werden.

### Anwendungsbeispiel 6b: Non-stick Kaugummi

Die Kaugummibase K1 bestand aus 2,0% Butyl Rubber (Isobuten-Isopren-Copolymer, MW 400000), 6,0% Polyisobuten (MW = 43800), 43,5% Polyvinylacetat (MW = 12000), 31,5% Polyvinylacetat (MW = 47000), 6,75% Triacetin und 10,25% Calciumcarbonat. Die Herstellung der Kaugummibase K1 und der Kaugummis kann analog zu US 5,601,858 erfolgen.

| | I (Gew.-%) | II (Gew.-%) | III (Gew.-%) |
|---|---|---|---|
| Kaugummibase K1 | 26,00 | 26,00 | 26,00 |
| Triacetin | 0,25 | 0,25 | 0,25 |
| Lecithin | 0,50 | 0,50 | 0,50 |
| Sorbitol, kristallin | 40,80 | 40,60 | 40,50 |
| Mannitol | 15,30 | 15,20 | 15,10 |
| Glycerin | 12,10 | 12,00 | 11,80 |
| Aspartam | 0,17 | 0,17 | 0,17 |
| Verkapseltes Aspartam | 1,08 | 1,08 | 1,08 |
| Amorphes Silica | 1,00 | 1,00 | 1,00 |
| Baumwollsamenöl | 0,50 | 0,50 | 0,50 |
| Polyoxyethylen-sorbitan-monolaurat (E 432) | 1,00 | 1,00 | 1,00 |
| Verkapseltes Spearmint-Aroma (enthält L-Carvon) | 0,20 | 0,10 | 0,30 |
| Verkapseltes Wintergrün- Aroma (enthält Methylsalicylat) | - | 0,40 | - |
| Pfefferminzöl | 0,80 | 0,80 | 1,00 |
| Aromakomposition D aus Anwendungsbeispiel 1 | 0,20 | 0,40 | 0,50 |
| L-Menthyl-L-lactat | 0,10 | - | 0,30 |

### Anwendungsbeispiel 6c: Bubble gum

Die Herstellung der Bubble Gums kann analog zu US 5,093,136 erfolgen.

| | I (Gew.-%) | II (Gew.-%) |
|---|---|---|
| Styrol-Butadien-Copolymer (SBR) | 19,50 | 17,50 |
| Polyisobuten | 8,00 | 8,00 |
| Sorbitol Pulver | 49,19 | 47,19 |
| Sorbitol, 70 %, in Wasser | 9,20 | 22,20 |
| Hydrierte Stärkehydrolysate (HSH) | 9,00 | - |
| Glycerin | 3,00 | 2,00 |
| Aspartam | 0,10 | 0,10 |
| Verkapseltes Aspartam | 0,50 | 0,50 |
| Roter und blauer Farbstoff | 0,01 | 0,01 |
| Erdbeer-Himbeer-Aroma | 1,00 | 2,20 |
| Aromakomposition A aus Anwendungsbeispiel 1 | 0,50 | 0,30 |

Die Kaugummis der Rezeptur (I) wurden als kompakte Kugeln, die der Rezeptur (II) als Hohlkugeln ausgeformt.

### Anwendungsbeispiel 6d: Kaugummi

Die Kaugummibase K2 bestand aus 28,5% Terpenharz, 33,9% Polyvinylacetat (MW = 14000), 16,25% hydriertes Pflanzenöl, 5,5% Mono- und Diglyceride, 0,5% Polyisobuten (MW 75000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis kann analog zu US 6,986,907 erfolgen.

| | I (Gew.-%) | II (Gew.-%) | III (Gew.-%) |
|---|---|---|---|
| Kaugummibase K2 | 25,30 | 27,30 | 26,30 |
| Sorbitol | 61,48 | 59,48 | 61,60 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,48 |
| Menthol, sprühgetrocknet | 1,00 | 0,50 | 0,40 |
| Kirscharoma, sprühgetrocknet | - | 1,20 | - |
| Citronen-Aroma | 1,00 | 1,00 | 1,48 |
| Orangenöl, natürlich | 0,80 | - | - |
| Aromakomposition B aus Anwendungsbeispiel 1 | 0,20 | | 0,20 |
| Aromakomposition A aus Anwendungsbeispiel 1 | | 0,30 | |

Die Kaugummis der Rezeptur (I) und (II) wurden als Streifen, die der Rezeptur (III) als Pellets ausgeformt.

### Anwendungswbeispiel 6e: Coffein-haltiqer Kaugummi

| **Teil** | **Inhaltsstoff** | **Einsatz in Gew.-%** |
|---|---|---|
| A | Kaugummibase, Company "Jagum T" | 30,70 |
| B | Sorbit, pulverisiert | 37,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Coffein | 4,00 |
| | Emulgum^{®} (Colloides Naturels, Inc.) | 0,30 |
| C | Sorbitol, 70%ige wässrige Lösung | 11,00 |
| | Glycerin | 1,00 |
| D | Krauseminzaroma | 0,80 |
| | Aromakomposition E aus Anwendungsbeispiel 2 | 0,50 |

Teile A bis D werden gemischt und intensiv geknetet. Die Rohmasse kann z.B. in Form von dünnen Streifen zu verzehrsfertigen Kaugummis verarbeitet werden.

### Anwendungsbeispiel 7: Zahnpasta

| **Teil** | **Inhaltsstoff** | **Einsatz in Gew.-%** |
|---|---|---|
| A | demineralisiertes Wasser | 22,00 |
| | Sorbitol, 70%ige wässrige Lösung | 45,00 |
| | Solbrol^{®} M, Natriumsalz (Bayer AG, p-Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Pfefferminzaroma | 0,80 |
| | Aromakomposition D aus Anwendungsbeispiel 1 | 0,20 |

Die Inhaltsstoffe der Teile A und B werden jeweils für sich vorgemischt und zusammen unter Vakuum bei 25 - 30°C 30 min gut verrührt. Teil C wird vorgemischt und zu A und B gegeben; D wird hinzugefügt und die Mischung unter Vakuum bei 25 - 30°C 30 min gut verrührt. Nach Entspannung ist die Zahnpasta fertig und kann abgefüllt werden.

### Anwendungsbeispiel 8: Zuckerreduzierte Erfrischungsgetränke

Vergleichszubereitung mit normalem Sucrosegehalt (A)

Vergleichszubereitung mit reduziertem Sucrosegehalt und Hesperetin und Phloretin (B)

Erfindungsgemäße Zubereitungen (C - H)

| | **Zubereitung (Einsatz in Gew.-%)** | | | | | | | |
|---|---|---|---|---|---|---|---|---|
| **Inhaltsstoff** | A | B | C | D | E | F | G | H |
| Wasser | 89,8 | 91,84 5 | 91,82 | 91,825 | 91,325 | 91,625 | 91,535 | 91,34 |
| Sucrose | 10,0 | 8,0 | 8,0 | 8,0 | 8,0 | 8,0 | 8,0 | 8,0 |
| Citronensäure | 0,2 | 0,15 | 0,15 | 0,15 | 0,15 | 0,15 | 0,15 | 0,15 |
| Fructose | | | | | | 0,2 | | |
| Tagatose | | | | | | | 0,3 | |
| Maltitol-Sirup | | | | | 0,5 | | | |
| Erythrit | | | | | | | | 0,5 |
| Hesperetin | - | 0,003 | | | | | | |
| Phloretin | - | 0,002 | | | | | | |
| Aromakomposition C aus Anwendungsbeispiel 2 | - | - | 0,03 | - | - | - | 0,015 | - |
| Aromakomposition B aus Anwendungsbeispiel 2 | - | - | - | 0,025 | 0,025 | 0,025 | - | 0,01 |

Die Stoffe wurden vorgelegt und mit Wasser aufgefüllt und gelöst. Die erfindungsgemäßen Zubereitungen C-H zeigten nicht nur eine ähnliche Gesamtsüße wie die Vergleichszubereitungen (A) und (B), sondern zusätzlich einen wesentlich verbesserten Profilverlauf, insbesondere bei der Anfangssüße.

### Anwendungsbeispiel 9: Fettarme Joghurts

Vergleichszubereitung mit Zucker (A)

Erfindungsgemäße Zubereitungen mit Süßstoffmischung und erfindungsgemäßen Aromakompositionen (B-D)

| | **Zubereitung (Angaben als Gew.-%)** | | | |
|---|---|---|---|---|
| **Inhaltsstoff** | A | B | C | D |
| Sucrose | 10 % | 8 % | 7 % | 6 % |
| Tagatose | - | - | | 0,5 % |
| Fructose | - | - | | 0,5 % |
| Aromakomposition A nach Anwendungsbeispiel 2 | - | 0,05 % | - | |
| Aromakomposition A nach Anwendungsbeispiel 1 | - | - | 0,4 % | - |
| Aromakomposition D nach Anwendungsbeispiel 1 | - | - | - | 0,4 % |
| Joghurt, 0,1 % Fett | zu 100 % auffüllen | zu 100 % auffüllen | zu 100 % auffüllen | zu 100 % auffüllen |

Die Inhaltsstoffe wurden gemischt und bei 5°C gekühlt.

### Anwendungsbeispiel 10: Verwendung zusammen mit Süßstoffen in fettarmen Joghurts

Vergleichszubereitung mit Süßstoffmischung (A)

Erfindungsgemäße Zubereitungen mit Süßstoffmischung und erfindungsgemäßen Aromakompositionen (B-D)

| | **Zubereitung (Angaben als Gew.-%)** | | | |
|---|---|---|---|---|
| **Inhaltsstoff** | A | B | C | D |
| D-Tagatose | 0,482 % | 0,482 % | 0,482 % | 0,482 % |
| Sucralose | 0,003 % | 0,003 % | 0,003 % | 0,003 % |
| Aspartam | 0,005 % | 0,005 % | 0,005 % | 0,005 % |
| Acesulfam K | 0,01 % | 0,01 % | 0,01 % | 0,01 % |
| Aromakomposition A nach Anwendungsbeispiel 2 | - | 0,05 % | - | - |
| Aromakomposition A nach Anwendungsbeispiel 1 | - | - | 0,4 % | - |
| Aromakomposition D nach Anwendungsbeispiel 1 | - | - | - | 0,4 % |
| Joghurt, 0,1 % Fett | zu 100 % auffüllen | zu 100 % auffüllen | zu 100 % auffüllen | zu 100 % auffüllen |

Die Inhaltsstoffe wurden gemischt und bei 5°C gekühlt.

### Anwendungsbeispiel 11: Milchmischgetränke

Vergleichszubereitungen mit Zucker (A-B)

Erfindungsgemäße Zubereitungen mit Zucker und erfindungsgemäßen Aromakompositionen (C-E)

| | **Zubereitung (Angaben als Gew.-%)** | | | | |
|---|---|---|---|---|---|
| **Inhaltsstoff** | **A** | **B** | **C** | **D** | **E** |
| Sucrose | 10,0 | 8,0 | 8,0 | 8,0 | 7,0 |
| Fructose | - | - | - | - | 0,5 |
| Tagatose | - | - | - | - | 0,5 |
| Aromakomposition A nach Anwendungsbeispiel 2 | - | - | 0,025 | - | - |
| Aromakomposition A nach Anwendungsbeispiel 1 | - | - | - | 0,2 | - |
| Aromakomposition D nach Anwendungsbeispiel 1 | - | - | - | - | 0,2 |
| H-Milch, 1,5 % Fett | zu 100 % auffüllen | | | | |

Die Inhaltsstoffe wurden gemischt, mit Milch aufgefüllt, gut gerührt, in Flaschen abgefüllt und bei 5°C gekühlt gelagert.

### Anwendungsbeispiel 12: Zuckerreduziertes Tomatenketchup

Vergleichszubereitung mit Zucker (A)

Vergleichszubereitung mit reduziertem Zuckeranteil (B)

Erfindungsgemäße Zubereitungen mit Zucker und erfindungsgemäßen Aromakompositionen (C-I)

| | **Zubereitung (Angaben als Gew.-%)** | | | | | | | |
|---|---|---|---|---|---|---|---|---|
| **Inhaltsstoff** | **A** | **B** | **C** | **E** | **F** | **G** | **H** | **I** |
| Kochsalz | 2 | 2 | 2 | 2 | 2 | 2 | 2 | 2 |
| Stärke, Farinex WM 55 | 1 | 1 | 1 | 1 | 1 | 1 | 1 | 1 |
| Sucrose | 12 | 9,6 | 9,2 | 8,4 | 9,6 | 9,6 | 8,4 | 8,4 |
| Tomaten-Konzentrat 2-fach | 40 | 40 | 40 | 40 | 30 | 30 | 30 | 30 |
| Glucosesirup 80 Brix | 18 | 18 | 18 | 18 | 18 | 18 | 18 | 18 |
| Branntweinessig 10% | 7 | 7 | 7 | 7 | 3 | 3 | 3 | 3 |
| Wasser | 20 | 22,4 | 22,4 | 23,2 | 36,0 | 36,0 | 37,2 | 37,2 |
| Aromakomposition A nach Anwendungsbeispiel 1 | | | 0,4 | | | 0,4 | | |
| Aromakomposition B nach Anwendungsbeispiel 1 | | | | | | | 0,4 | |
| Aromakomposition D nach Anwendungsbeispiel 1 | | | | 0,4 | 0,4 | | | 0,4 |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 13: Zuckerreduzierte Eiscremes

Vergleichszubereitung mit Zucker (A)

Vergleichszubereitung mit reduziertem Zuckeranteil (B)

Erfindungsgemäße Zubereitungen mit Zucker und erfindungsgemäßen Aromakompositionen (C-F)

| | **Zubereitung (Gehalt in Gew.-%)** | | | | | |
|---|---|---|---|---|---|---|
| **Inhaltsstoff** | **A** | **B** | **C** | **D** | **E** | **F** |
| Magermilch | 57,15 | 61,15 | 60,95 | 61,05 | 60,95 | 61,05 |
| Pflanzenfett, Schmelzbereich 35 - 40 °C | 20,00 | 20,00 | 20,00 | 20,00 | 20,00 | 20,00 |
| Zucker (Saccharose) | 12,00 | 8,00 | 8,00 | 8,00 | 8,00 | 8,00 |
| Magermilchpulver | 5,00 | 5,00 | 5,00 | 5,00 | 5,00 | 5,00 |
| Glucosesirup 72 % Trockensubstanz | 5,00 | 5,00 | 5,00 | 5,00 | 5,00 | 5,00 |
| Emulgator SE 30 (Grindstedt Products, Dänemark) | 0,65 | 0,65 | 0,65 | 0,65 | 0,65 | 0,65 |
| Aroma, enthaltend 0,1 % Diacetyl und 1 % Vanillin | 0,20 | 0,20 | 0,20 | 0,20 | | |
| Aroma, enthaltend 0,1 % Diacetyltrimer nach WO 2006/058893, 0,1 % Diacetyl und 1 % Vanillin | | | | | 0,20 | 0,20 |
| Aroma nach Anwendungsbeispiel 4, Zubereitung E | | | 0,20 | | 0,20 | |
| Aroma nach Anwendungsbeispiel 4, Zubereitung H | | | | 0,10 | | 0,10 |

Magermilch und Glucosesirup wurden auf 55° erhitzt und Zucker, Magermilchpulver sowie Emulgator hinzugefügt. Das Pflanzenfett wurde vorerhitzt und die gesamte Masse auf 58°C erwärmt. Nach Zugabe des Aromas wurde mit Hilfe eines Durchflusshochdruckhomogenisators homogenisiert (180 / 50 bar). Die erhaltene Masse wurde 1 min lang bei 78°C temperiert, anschließend auf 2 - 4 °C abgekühlt und 10 h zur Reifung bei dieser Temperatur inkubiert. Danach wurde die gereifte Masse abgefüllt und bei -18°C eingefroren gelagert.

### Anwendungsbeispiel 14: Für Diabetiker geeignetes Eis

Es wurde ein für Diabetiker geeignetes Eis aus folgenden Zutaten hergestellt und in Portionen zu je 95 mL in Becher abgefüllt:
Eingedickte entrahmte Milch, Fructosesirup, Erdbeerstücke und Erdbeerpüree (15%), Pflanzenfett, Diätschokoladensplitter (3,5%, mit Emulgator Sojalecithin), Molkenerzeugnis, Rote-Bete-Saft, Johannisbrotkernmehl, Guarkernmehl, Carrageen, Emulgator (E 471), Gelatine, Säuerungsmittel Citronensäure, Erdbeer-Aroma (enthaltend 40 Gew.-% Aromakomposition A nach Anwendungsbeispiel 1), bezogen auf das Gesamtgewicht des Erdbeer-Aromas), Farbstoff Carotin.

### Nährwert (pro 95 mL):

Eiweiss 1,8 g, Kohlenhydrate 13,3 g (davon Fructose 9,5 g), Fett 4,2 g.

### Anwendungsbeispiel 15: Diätschokolade auf Basis von Maltit

Es wurde eine für Diabetiker geeignete Schokolade aus folgenden Zutaten hergestellt und in rechteckige Tafeln gegossen:
Maltit, Haselnussmasse, Kakaobutter, Magermilchpulver, Kakaomasse, Inulin, Butterreinfett, Emulgator Sojalecithine, Vanille-Aroma (enthaltend Vanilleschoten-Extrakt, Vanillin und 40 Gew.-% Aromakomposition D nach Anwendungsbeispiel 1, bezogen auf das Gesamtgewicht des Vanille-Aromas).

### Nährwert (pro 100 g):

Eiweiss 8 g, Kohlenhydrate 43 g (davon Maltit 34 g), Fett 34 g.

### Anwendungsbeispiel 16: Diätschokolade auf Basis von Fructose

Es wurde eine für Diabetiker geeignete Schokolade aus folgenden Zutaten hergestellt und in rechteckige Tafeln gegossen:
Kakaomasse, Fructose, Magermilchpulver, Kakaobutter, Inulin, Butterreinfett, Emulgator Sojalecithin, Walnüsse, Speisesalz, Vanille-Aroma (enthaltend Vanillin und 40 Gew.-% Aromakomposition A nach Anwendungsbeispiel 1, bezogen auf das Gesamtgewicht des Vanille-Aromas).

### Nährwert (pro 100 g):

Eiweiss 8,8 g, Kohlenhydrate 34 g (davon Fructose 23 g, Lactose 7,5 g, Saccharose 1,4 g), Fett 36 g; Ballaststoffe 18,5 (davon 12,2 g Inulin); Natrium: 0,10 g. Kakao-Anteil mindestens 50 Gew.-%.

### Anwendungsbeispiel 17: Zuckerreduzierte Müslimischung

| Nr. | | A (Vergleich) (Gew.-%) | B (erfindungsgemäß; zuckerreduziert) (Gew.-%) |
|---|---|---|---|
| 1 | Haferflocken | 17,00 | 18,90 |
| 2 | Knusprige Haferflocken-Cluster | 10,00 | 12,00 |
| 3 | Reis-Crispies | 16,90 | 17,80 |
| 4 | Cornflakes | 16,50 | 17,50 |
| 5 | Korinthen | 3,50 | 3,50 |
| 6 | Haselnüsse, zerhackt | 2,50 | 2,50 |
| 7 | Glucose-Sirup aus Weizen, DE 30 | 9,50 | 9,50 |
| 8 | Saccharose | 20,00 | 14,00 |
| 9 | Wasser | 4,00 | 4,00 |
| 10 | Citronensäurepulver, wasserfrei | 0,10 | 0,10 |
| 11 | Aromakomposition D nach Anwendungsbeispiel 1 | - | 0,20 |

Jeweils Bestandteile Nr. 1 bis 6 in einer Drehtrommel mischen (Mix 1). Jeweils Bestandteile Nr. 7 bis 9 erwärmen und Bestandteil Nr. 10 zugeben (sowie in Rezeptur B zusätzlich den Bestandteil Nr. 11 zugeben) (Mix 2). Jeweils Mix 2 zu Mix 1 geben und gut mischen. Zuletzt die resultierende Müslimischung auf ein Backblech geben und in einem Ofen bei 130°C für 8 Minuten trocknen.

### Anwendungsbeispiel 18: Zuckerreduzierte Fruchtgummis

| | A (Vergleich) (Gew.-%) | B (erfindungsgemäß; zuckerreduziert) (Gew.-%) |
|---|---|---|
| Wasser | 23,70 | 25,60 |
| Saccharose | 34,50 | 8,20 |
| Glucosesirup, DE 40 | 31,89 | 30,09 |
| Iso Syrup C* Tru Sweet 01750 (Cerestar GmbH) | 1,50 | 2,10 |
| Gelatine 240 Bloom | 8,20 | 9,40 |
| Polydextrose (Litesse^{®} Ultra, Danisco Cultor GmbH) | - | 24,40 |
| Gelber und roter Farbstoff | 0,01 | 0,01 |
| Citronensäure | 0,20 | |
| Aroma nach Anwendungsbeispiel 4, Zubereitung F | - | 0,20 |

Polydextrose ist ein selbst nicht süß schmeckendes Polysaccharid mit niedrigem Brennwert.

### Anwendungsbeispiel 19: Schoko-Cappuccino-Eiscreme

| | A (Gew.-%) | B (Gew.-%) |
|---|---|---|
| Glucose-Fructose-Sirup | 14,10 | 14,10 |
| Saccharose | 10,00 | 7,50 |
| Magermilchpulver | 5,00 | 5,00 |
| Sahne (36% Fettanteil) | 24,00 | 24,00 |
| Emulgator und Stabilisator Cremodan^{®} 709VEG (Danisco) | 0,50 | 0,50 |
| Kakaopulver | 5,975 | 5,975 |
| Carrageenan | 0,025 | 0,025 |
| Wasser | 40,20 | 42,50 |
| Cappuccino-Aroma | 0,20 | 0,20 |
| Aromakomposition A nach Anwendungsbeispiel 1 | - | 0,20 |

### Anwendungsbeispiel 20: Gelatinekapseln zum Direktverzehr

| | I (Gew.-%) | II (Gew.-%) | III (Gew.-%) |
|---|---|---|---|
| Gelatinehülle: | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |
| | | | |
| Kernzusammensetzung: | | | |
| Pflanzenöl-Triglycerid (Kokosöl - Fraktion) | 79,49 | 68,55 | 58,55 |
| Orangen-Aroma | 8,0 | 16,0 | 23,65 |
| Aromakomposition A nach Anwendungsbeispiel 1 | 2,0 | 4,0 | 5,0 |
| Neotam und Aspartam | 0,01 | 0,05 | - |
| Sucralose | 0,10 | 0,15 | 0,40 |
| 2-Hydroxypropylmenthylcarbonat | 0,33 | 0,20 | - |
| 2-Hydroxyethylmenthylcarbonat | - | 0,20 | 1,00 |
| (1 R,3R,4S) Menthyl-3-carbonsäure-N-ethylamid (WS-3) | - | 0,55 | 0,50 |
| (-)-Menthonglycerinacetal (Frescolat MGA) | - | 0,30 | 0,80 |
| Vanillin | 0,07 | - | 0,10 |

Die zum Direktverzehr geeigneten Gelatinekapseln wurden gemäß WO 2004/050069 hergestellt und hatten einen Durchmesser von 5 mm, das Gewichtsverhältnis von Kernmaterial zu Hüllmaterial lag bei 90 : 10. Die Kapseln öffneten sich im Mund innerhalb von weniger als 10 Sekunden und lösten sich vollständig innerhalb von weniger als 50 Sekunden auf.

## Patentansprüche

1. Aromakomposition, bestehend aus oder enthaltend eine Substanz (i) sowie entweder
- eine Substanz (ii) oder
- eine Substanz (iii) oder
- eine Mischung von Substanzen (ii) und (iii), wobei gilt:
(i) besteht aus einem oder mehreren Alkamiden ausgewählt aus der Gruppe bestehend aus:
2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,4E-Decadiensäure-N-([2S]-2-methylbutyl)amid, 2E,4E-Decadiensäure-N-([2S]-2-methylbutyl)amid, 2E,4E-Decadiensäure-N-([2R]-2-methylbutylamid), 2E,4Z-Decadiensäure-N-(2-methylbutyl)amid, 2E,4E-Decadiensäure-N-piperid (Achilleamid), 2E,4E-Decadiensäure-N-piperid (Sarmentin), 2E-Decensäure-N-isobutylamid, 3E-Decensäure-N-isobutylamid, 3E-Nonensäure-N-isobutylamid, 2E,6Z,8E-Decatriensäure-N-isobutylamid (Spilanthol), 2E,6Z,8E-Decatriensäure-N-([2S]-2-methylbutyl)amid (Homospilanthol), 2E,6Z,8E-Decatriensäure-N-([2R]-2-methylbutyl)amid, 2E-Decen-4-insäure-N-isobutylamid, 2Z-Decen-4-insäure-N-isobutylamid, 2E,6Z,8E,10E-Dodecatetraensäure-N-(2-methylpropyl)amid (α-Sanshool), 2E,6Z,8E,10E-Dodecatetraensäure-N-(2-hydroxy-2-methylpropyl)amid (α-Hydroxysanshool), 2E,6E,8E,10E-Dodecatetraensäure-N-(2-hydroxy-2-methylpropyl)amid (β-Hydroxysanshool), 2E,4E,8Z,10E,12E-Tetradecapentaensäure-N-(2-hydroxy-2-methylpropyl)amid (γ-Hydroxysanshool), 2E,4E,8E,10E,12E-Tetradecapentaensäure-N-(2-hydroxy-2-methylpropyl)amid (y-Hydroxyisosanshool), 2E,4E,8Z,10E,12E-Tetradecapentaensäure-N-(2-methyl-2-propenyl)amid (γ-dehydrosanshool), 2E,4E,8Z,10E,12E-Tetradecapentaensäure-N-(2-methylpropyl)amid (γ-Sanshool), 2E,4E,8Z,11Z-Tetradecatetraensäure-N-(2-hydroxy-2-methylpropyl)amid (Bungeanool), 2E,4E,8Z,11E-Tetradecatetraensäure-N-(2-hydroxy-2-methylpropyl)amid (Isobungeanool), 2E,4E,8Z-Tetradecatriensäure-N-(2-hydroxy-2-methylpropyl)amid (Dihydrobungeanool) und 2E,4E -Tetradecadiensäure-N-(2-hydroxy-2-methylpropyl)amid (Tetrahydrobungeanool);
(ii) ist Hesperetin der Formel (I) 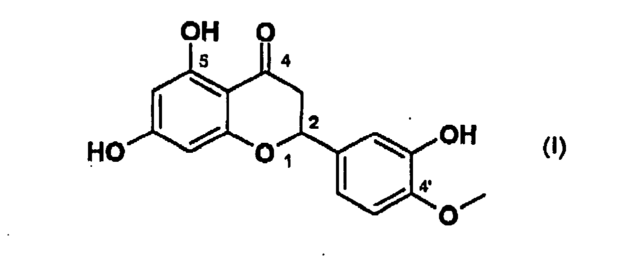 wobei das Hesperetin der Formel (I) als (2S)-Enantiomer, (2R)-Enantiomer oder beliebige Mischung der beiden Enantiomere vorliegt, und/oder dessen Salzen
und
(iii) ist
- ein 4-Hydroxydihydrochalkon der Formel (II) 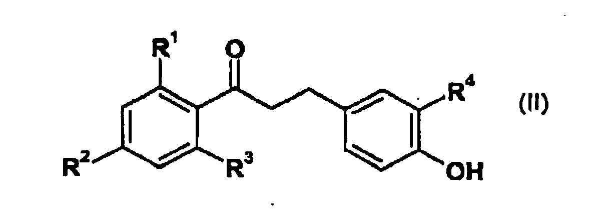 wobei
R¹, R², R³ und R⁴ unabhängig voneinander jeweils H, OH oder O-Alkyl bedeuten, mit der Maßgabe, dass mindestens einer der Reste R¹, R² oder R³ OH bedeutet,
- ein Salz eines solchen 4-Hydroxydihydrochalkons der Formel (II),
- eine Mischung umfassend oder bestehend aus zwei oder mehr unterschiedlichen 4-Hydroxydihydrochalkonen der Formel (II), wobei R¹, R², R³ und R⁴ jeweils die oben angegebene Bedeutung haben,
- eine Mischung umfassend oder bestehend aus Salzen von zwei oder mehr unterschiedlichen 4-Hydroxydihydrochalkonen der Formel (II), wobei R¹, R², R³ und R⁴ jeweils die oben angegebene Bedeutung haben
oder
- eine Mischung umfassend oder bestehend aus einem 4-Hydroxydihydrochalkon der Formel (II) oder zwei oder mehr unterschiedlichen 4-Hydroxydihydrochalkonen der Formel (II), wobei R¹, R², R³ und R⁴ jeweils die oben angegebene Bedeutung haben, und einem Salz eines 4-Hydroxydihydrochalkons der Formel (II) oder zwei oder mehr Salzen von unterschiedlichen 4-Hydroxydihydrochalkonen der Formel (II), wobei R¹, R², R³ und R⁴ jeweils die oben angegebene Bedeutung haben.

2. Aromakomposition nach Anspruch 1, wobei gilt
(i) besteht aus einem oder mehreren Alkamiden ausgewählt aus der Gruppe bestehend aus:
2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,4E-Decadiensäure-N-piperid (Achilleamid), 2E,6Z,8E-Decatriensäure-N-isobutylamid (Spilanthol), 2E,6Z,8E-Decatriensäure-N-([2S]-2-methylbutyl)amid (Homospilanthol), 2E,6Z,8E-Decatriensäure-N-([2R]-2-methylbutyl)amid, 2E,6Z,8E,10E-Dodecatetraensäure-N-(2-methylpropyl)amid (α-Sanshool), 2E,6Z,8E,10E-Dodecatetraensäure-N-(2-hydroxy-2-methylpropyl)amld (α-Hydroxysanshool), 2E,4E,8Z,10E,12E-Tetradecapentaensäure-N-(2-methylpropyl)amid (γ-Sanshool) und 2E,4E,8Z,11Z-Tetradecatetraensäure-N-(2-hydroxy-2-methylpropyl)amid (Bungeanool);
und/oder
(ii) ist Hesperetin der Formel (I) 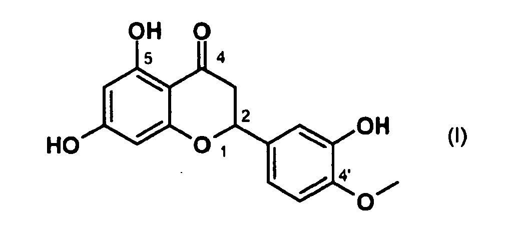 wobei das Hesperetin der Formel (I) als (2S)-Enantiomer, (2R)-Enantiomer oder beliebige Mischung der beiden Enantiomere vorliegt,
und/oder
(iii) ist ein 4-Hydroxydihydrochalkon der Formel (II), wobei in der Formel (II) jeweils R¹ OH bedeutet
R² und R³, unabhängig voneinander, H oder OH bedeuten,
und
R⁴ H oder Methoxy (OCH₃) bedeutet.

3. Aromakomposition nach einem der vorangehenden Ansprüche, wobei gilt:
(i) besteht aus einem oder mehreren Alkamiden ausgewählt aus der Gruppe bestehend aus:
2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,62,8E-Decatriensäure-N-isobutylamid (Spilanthol), 2E,6Z,8E,10E-Dodecatetraensäure-N-(2-methylpropyl)amid (α-Sanshool), 2E,4E,8Z,11Z-Tetradecatetraensäure-N-(2-hydroxy-2-methylpropyl)amid (Bungeanool);
und/oder
(ii) ist Hesperetin der Formel (I) 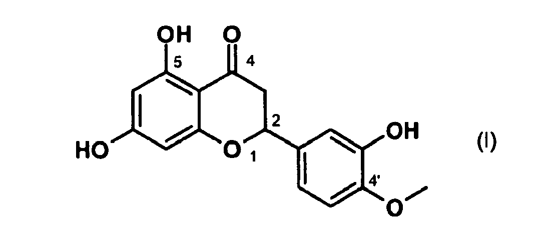 wobei das Hesperetin der Formel (I) als (2S)-Enantiomer oder beliebige Mischung der beiden Enantiomere vorliegt,
und/oder
(iii) besteht aus
- einem 4-Hydroxydihydrochalkon der Formel (II) ausgewählt aus der Gruppe bestehend aus:
3-(4-Hydroxyphenyl)-1-(2-hydroxyphenyl)propan-1-on (2',4-Dihydroxydihydrochalkon; Verbindung 1),
3-(4-Hydroxyphenyl)-1-(2,4-dihydroxyphenyl)propan-1-on (2',4,4'-Trihydroxydihydrochalkon; Davidigenin; Verbindung 2),
3-(4-Hydroxyphenyl)-1-(2,6-dihydroxyphenyl)propan-1-on (2',4,6'-Trihydroxydihydrochalkon; Verbindung 3),
3-(4-Hydroxyphenyl)-1-(2,4,6-trihydroxyphenyl)propan-1-on (2',4,4',6'-Tetrahydroxydihydrochalkon; Phloretin; Verbindung 4), 3-(4-Hydroxy-3-methoxyphenyl)-1-(2-hydroxyphenyl)propan-1-on (2',4-Dihydroxy-3-methoxydihydrochalkon; Verbindung 5),
3-(4-Hydroxy-3-methoxyphenyl)-1-(2,4-dihydroxyphenyl)propan-1-on (2',4,4'-Trihydroxy-3-methoxydihydrochalkon; Verbindung 6),
3-(4-Hydroxy-3-methoxyphenyl)-1-(2,6-dihydroxyphenyl)propan-1-on (2',4,6'-Trihydroxy-3-methoxydihydrochalkon; Verbindung 7),
3-(4-Hydroxy-3-methoxyphenyl)-1-(2,4,6-trihydroxyphenyl)propan-1-on (2',4,4',6'-Tetrahydroxy-3-methoxydihydrochalkon; Verbindung 8) und
3-(3,4-Dihydroxyphenyl)-1-(2,4,6-trihydroxyphenyl)propan-1-on (2',3,4,4',6'-Tetrahydroxydihydrochalkon; Verbindung 9),
- einem Salz eines solchen 4-Hydroxydihydrochalkons der Formel (II),
- einer Mischung umfassend oder bestehend aus zwei oder mehr aus der besagten Gruppe ausgewählten 4-Hydroxydihydrochalkonen der Formel (II).
- eine Mischung umfassend oder bestehend aus Salzen von zwei oder mehr aus der besagten Gruppe ausgewählten 4-Hydroxydihydrochalkonen der Formel (II)
oder
- einer Mischung umfassend oder bestehend aus
einem der aus der besagten Gruppe ausgewählten 4-Hydroxydihydrochalkone der Formel (II) oder zwei oder mehr aus der besagten Gruppe ausgewählten 4-Hydroxydihydrochalkonen der Formel (II) und
einem Salz eines aus der besagten Gruppe ausgewählten 4-Hydroxydihydrochalkons der Formel (II) oder zwei oder mehr Salzen von unterschiedlichen aus der besagten Gruppe ausgewählten 4-Hydroxydihydrochalkonen der Formel (II).

4. Aromakomposition nach einem der vorangehenden Ansprüche, wobei gilt:
(i) besteht aus einem oder mehreren Alkamiden ausgewählt aus der Gruppe bestehend aus:
2E,4E-Decadiensäure-N-isobutylamid (Pellitorin), 2E,6Z,8E-Decatriensäure-N-isobutylamid (Spilanthol), 2E,6Z,8E,10E-Dodecatetraensäure-N-(2-methylpropyl)amid (α-Sanshool) und 2E,4E,8Z,11Z-Tetradecatetraensäure-N-(2-hydroxy-2-methylpropyl)amid (Bungeanool);
und/oder
(ii) ist Hesperetin der Formel (I) 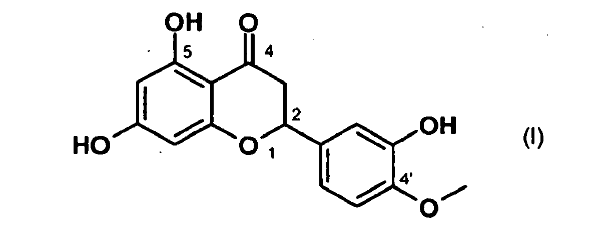 wobei das Hesperetin der Formel (I) als (2S)-Enantiomer oder beliebige Mischung der beiden Enantiomere vorliegt,
und/oder
(iii) besteht aus oder umfasst Phloretin (Verbindung 4).

5. Aromakomposition nach einem der vorangehenden Ansprüche, wobei
- das Gewichtsverhältnis der Gesamtmenge von Substanzen (i) zu der Gesamtmenge von Substanzen (ii) und (iii) in einem Bereich von 1:1000000 bis 1:1 liegt, bevorzugt in einem Bereich von 1:10000 bis 1:10, besonders bevorzugt in einem Bereich von 1:2000 bis 1:50
und/oder
- das Gewichtsverhältnis der Gesamtmengen von Substanzen (ii) zu Substanzen (iii) im Bereich von 1:10 bis 10:1 liegt, besonders bevorzugt im Bereich von 5:1 bis 1:5 und insbesondere bevorzugt im Bereich von 7:3 bis 3:7.

6. Verwendung einer Aromakomposition nach einem der vorangehenden Ansprüche
- zur Verstärkung des süßen Geschmacks eines süß schmeckenden Stoffes oder des süßen Geruchseindrucks eines Aromastoffes, der einen süßen Geruchseindruck verursacht
oder
- zur Verstärkung der Anfangssüße eines süß schmeckenden Stoffes oder eines Aromastoffes, der einen süßen Geruchseindruck verursacht.

7. Zubereitung aus der Gruppe bestehend aus der Ernährung, der Mundpflege oder dem Genuss dienenden Zubereitungen, Halbfertigwaren, Riech-, Aroma- oder Geschmackstoffkompositionen oder Würzmischungen, umfassend die folgenden Komponenten:
(a) eine Aromakomposition nach einem der vorangehenden Ansprüche 1 bis 5 sowie zusätzlich
(b) einen oder mehrere süß schmeckende Stoffe
und/oder
(c) einen oder mehrere Aromastoffe, die einen süßen Geruchseindruck verursachen,
wobei die Gesamtmenge an Komponente (a) in der Zubereitung ausreicht, den süßen Geschmackseindruck des bzw. der süß schmeckenden Stoffe (b) bzw. den süßen Geruchseindruck des bzw. der Aromastoffe (c), die einen süßen Geruchseindruck verursachen, sensorisch zu verstärken.

8. Zubereitung nach Anspruch 7, umfassend als Komponente (b) einen oder mehrere Zucker, wobei die Gesamtmenge an Komponente (a) in der Zubereitung ausreicht, um im Vergleich mit einer Zubereitung oder Halbfertigware, die bei ansonsten identischer Zusammensetzung keine Komponente (a) aber zumindest die 1,05-fache Menge an Zucker umfasst, die gleiche oder eine verstärkte Anfangssüße zu vermitteln.

9. Zubereitung nach einem der Ansprüche 7 oder 8, umfassend
(b) einen oder mehrere weitere süß schmeckende Stoffe, wobei der oder die weiteren süß schmeckenden Stoffe ausgewählt sind aus der Gruppe bestehend aus:
- ein oder mehrere Kohlenhydrate ausgewählt aus der Gruppe bestehend aus Sucrose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrin und pflanzliche Zubereitungen enthaltend einen oder mehrere der genannten Kohlenhydrate,
- ein oder mehrere Zuckeralkohole ausgewählt aus der Gruppe bestehend aus Glycerin, Erythritol, Threitol, Arabitol, Ribitol, Xylitol, Sorbitol, Mannitol, Malitol, Isomaltit, Dulcitol und Lactitol,
- ein oder mehrere Proteine und/oder Aminosäuren aus der Gruppe bestehend aus Miraculin, Monellin, Thaumatin, Curculin, Brazzein, Glycin, D-Leucin, D-Threonin, D-Asparagin, D-Phenylalanin, D-Tryptophan, L-Prolin,
- ein oder mehrere Süßstoffe aus der Gruppe bestehend aus Magap, Natriumcyclamat, Acesulfam K, Neohesperidindihydrochalkon, Saccharin-Natriumsalz, Aspartam, Superaspartam, Neotam, Alitam, Sucralose, Stevioside, Rebaudioside, Lugduname, Carrelame, Sucrononate, Sucrooctate, Monatin und Phyllodulcin,
und deren Mischungen
und/oder
(c) einen oder mehrere Aromastoffe, die einen süßen Geruchseindruck verursachen, wobei der oder die weiteren Aromastoffe, die einen süßen Geruchseindruck verursachen, ausgewählt sind aus der Gruppe bestehend aus:
Vanillin, Ethylvanillin, Ethylvanillinisobutyrat (= 3-Ethoxy-4-isobutyryloxybenzaldehyd), Furaneol (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd.

10. Der Ernährung, der Mundpflege oder dem Genuss dienende Zubereitung nach einem der Ansprüche 7 bis 9, umfassend eine Gesamtmenge im Bereich von 0,1 bis 150 ppm, bevorzugt im Bereich von 1 bis 50 ppm, besonders bevorzugt im Bereich von 10 bis 50 ppm, an einer Aromakomposition nach einem der Ansprüche 1 bis 4, bezogen auf das Gesamtgewicht der Zubereitung.

11. Der Ernährung, der Mundpflege oder dem Genuss dienende Zubereitung nach einem der Ansprüche 7 bis 10, wobei die Gesamtmenge
- an Substanzen (i) im Bereich von 0,005 bis 5 ppm, bevorzugt 0,02 bis 2 ppm, besonders bevorzugt 0,05 bis 0,5 ppm liegt,
- an Substanzen (ii) im Bereich von 0,5 bis 500 ppm, bevorzugt 10 bis 200 ppm, besonders bevorzugt 20 bis 100 ppm liegt
und/oder
- an Substanzen (iii) im Bereich von 0,5 bis 500 ppm, bevorzugt 5 bis 100 ppm, besonders bevorzugt 10 bis 40 ppm liegt,
wobei die Gesamtmenge sämtlicher Komponenten (i), (ii) und (iii) im Bereich von 0,5 bis 500 ppm, bevorzugt im Bereich von 5 bis 200 ppm, besonders bevorzugt im Bereich von 10 bis 100 ppm liegt.

12. Der Ernährung, der Mundpflege oder dem Genuss dienende Zubereitung nach einem der Ansprüche 7 bis 11, wobei die Zubereitung ausgewählt ist aus der Gruppe bestehend aus:
(A) Süßwaren,
(B) alkoholische oder nicht-alkoholische Getränke oder Instantgetränke,
(C) Getreideprodukte und/oder Nussprodukte,
(D) Milchprodukte,
(E) Produkte aus Sojaprotein oder anderen Sojabohnen-Fraktionen,
(F) Frucht- und/oder Gemüsezubereitungen,
(G) Produkte auf Fett- und Ölbasis oder Emulsionen derselben,
(H) Mundpflegeprodukte.

13. Zubereitung nach einem der Ansprüche 7 bis 12, ausgewählt, aus der Gruppe bestehend aus Halbfertigwaren, Riech-, Aroma- oder Geschmacksstoffkompositionen oder Würzmischungen, umfassend eine Gesamtmenge im Bereich von 0,0001 Gew.-% bis 95 Gew.%, bevorzugt 0,001 Gew.% bis 80 Gew.-%, besonders bevorzugt 0,001 Gew.% bis 50 Gew.%, an einer Aromakomposition nach einem der Ansprüche 1 bis 5, bezogen auf das Gesamtgewicht der Zubereitung.

14. Verfahren zum Verstärken des süßen Geschmacks bzw. der Anfangssüße eines süß schmeckenden Stoffes oder des süßen Geruchseindruckes bzw. der Anfangssüße eines Aromastoffes, der einen süßen Geruchseindruck verursacht, mit folgendem Schritt:
- Mischen eines oder mehrerer süß schmeckender Stoffe (Komponente (b)) oder eines oder mehrerer Aromastoffe, der beziehungsweise die einen süßen Geruchseindruck verursachen (Komponente (c)), mit einer Gesamtmenge einer erfindungsgemäßen Aromakomposition (Komponente (a)) nach einem der Ansprüche 1 bis 5, wobei die Gesamtmenge an erfindungsgemäßer Aromakomposition (Komponente (a)) in der Zubereitung ausreicht, den süßen Geschmackseindruck des bzw. der süß schmeckenden Stoffe (b) bzw. den süßen Geruchseindruck des bzw. der Aromastoffe (c), die einen süßen Geruchseindruck verursachen, und insbesondere die Anfangssüße sensorisch zu verstärken.

15. Verwendung von einem oder mehreren Alkamiden (i) ausgewählt aus der Gruppe bestehend aus:
2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,4E-Decadiensäure-N-([2S]-2-methylbutyl)amid, 2E,4E-Decadiensäure-N-([2S]-2-methylbutyl)amid, 2E,4E-Decadiensäure-N-([2R]-2-methylbutylamid), 2E,4Z-Decadiensäure-N-(2-methylbutyl)amid, 2E,4E-Decadiensäure-N-piperid (Achilleamid), 2E,4E-Decadiensäure-N-piperid (Sarmentin), 2E-Decensäure-N-isobutylamid, 3E-Decensäure-N-isobutylamid, 3E-Nonensäure-N-isobutylamid, 2E,6Z,8E-Decatriensäure-N-isobutylamid (Spilanthol), 2E,6Z,8E-Decatriensäure-N-([2S]-2-methylbutyl)amid (Homospilanthol), 2E,6Z,8E-Decatriensäure-N-([2R]-2-methylbutyl)amid, 2E-Decen-4-insäure-N-isobutylamid, 2Z-Decen-4-insäure-N-isobutylamid, 2E,6Z,8E,10E-Dodecatetraensäure-N-(2-methylpropyl)amid (α-Sanshool), 2E,6Z,8E,10E-Dodecatetraensäure-N-(2-hydroxy-2-methylpropyl)amid (α-Hydroxysanshool), 2E,6E,8E,10E-Dodecatetraensäure-N-(2-hydroxy-2-methylpropyl)amid (β-Hydroxysanshool), 2E,4E,8Z,10E,12E-Tetradecapentaensäure-N-(2-hydroxy-2-methylpropyl)amid (γ-Hydroxysanshool), 2E,4E,8E,10E,12E-Tetradecapentaensäure-N-(2-hydroxy-2-methylpropyl)amid (γ-Hydroxyisosanshool), 2E,4E,8Z,10E,12E-Tetradecapentaensäure-N-(2-methyl-2-propenyl)amid (γ-dehydrosanshool), 2E,4E,8Z,10E,12E-Tetradecapentaensäure-N-(2-methylpropyl)amid (γ-Sanshool), 2E,4E,8Z,11Z-Tetradecatetraensäure-N-(2-hydroxy-2-methylpropyl)amid (Bungeanool), 2E,4E,8Z,11E-Tetradecatetraensäure-N-(2-hydroxy-2-methylpropyl)amid (Isobungeanool), 2E,4E,8Z-Tetradecatriensäure-N-(2-hydroxy-2-methylpropyl)amid (Dihydrobungeanool) und 2E,4E -Tetradecadiensäure-N-(2-hydroxy-2-methylpropyl)amid (Tetrahydrobungeanool);
zur Verstärkung der Anfangssüße einer Zubereitung umfassend
(ii) Hesperetin der Formel (I) 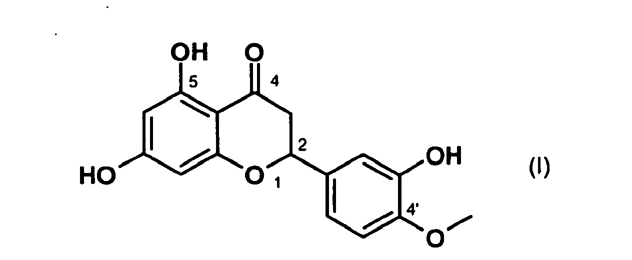 wobei das Hesperetin der Formel (I) als (2S)-Enantiomer, (2R)-Enantiomer oder beliebige Mischung der beiden Enantiomere vorliegt, und/oder dessen Salzen
und/oder
(iii)
- ein 4-Hydroxydihydrochalkon der Formel (II) 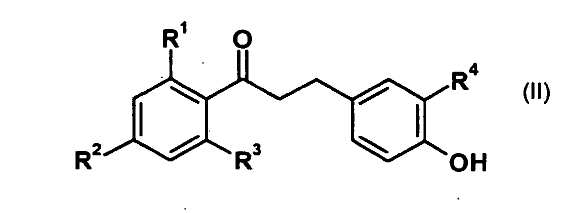 wobei
R¹, R², R³ und R⁴ unabhängig voneinander jeweils H, OH oder O-Alkyl bedeuten, mit der Maßgabe, dass mindestens einer der Reste R¹, R² oder R³ OH bedeutet,
- ein Salz eines solchen 4-Hydroxydihydrochalkons der Formel (II),
- eine Mischung umfassend oder bestehend aus zwei oder mehr unterschiedlichen 4-Hydroxydihydrochalkorien der Formel (II), wobei R¹, R², R³ und R⁴ jeweils die oben angegebene Bedeutung haben,
- eine Mischung umfassend oder bestehend aus Salzen von zwei oder mehr unterschiedlichen 4-Hydroxydihydrochalkonen der Formel (II), wobei R¹, R², R³ und R⁴ jeweils die oben angegebene Bedeutung haben
oder
- eine Mischung umfassend oder bestehend aus einem 4-Hydroxydihydrochalkon der Formel (II) oder zwei oder mehr unterschiedlichen 4-Hydroxydihydrochalkonen der Formel (II), wobei R¹, R², R³ und R⁴ jeweils die oben angegebene Bedeutung haben, und
einem Salz eines 4-Hydroxydihydrochalkons der Formel (II) oder zwei oder mehr Salzen von unterschiedlichen 4-Hydroxydihydrochalkonen der Formel (II), wobei R¹, R², R³ und R⁴ jeweils die oben angegebene Bedeutung haben,
sowie umfassend
(b) einen oder mehrere süß schmeckende Stoffe
(c) einen oder mehrere Aromastoffe, die einen süßen Geruchseindruck verursachen.

## Claims

1. Aroma composition consisting of or containing a substance (i) and either
- a substance (ii) or
- a substance (iii) or
- a mixture of substances (ii) and (iii), wherein:
(i) consists of one or more alkamides chosen from the group consisting of: I
2E,4E-decadienoic acid N-isobutylamide (pellitorine), 2E,4Z-decadienoic acid N-isobutylamide (cis-pellitorine), 2Z,4Z-decadienoic acid N-isobutylamide, 2Z,4E-decadienoic acid N-isobutylamide, 2E,4E-decadienoic acid N-([2S]-2-methylbutyl)amide, 2E,4E-decadienoic acid N-([2S]-2-methylbutyl)amide, 2E,4E-decadienoic acid N-([2R]-2-methylbutyl)amide, 2E,4Z-decadienoic acid N-(2-methylbutyl)amide, 2E,4E-decadienoic acid N-piperide (achilleamide), 2E,4E-decadienoic acid N-piperide (sarmentine), 2E-decenoic acid N-isobutylamide, 3E-decenoic acid N-isobutylamide, 3E-nonenoic acid N-isobutylamide, 2E,6Z,8E-decatrienoic acid N-isobutylamide (spilanthol), 2E,6Z,8E-decatrienoic acid N-([2S]-2-methylbutyl)amide (homospilanthol), 2E,6Z,8E-decatrienoic acid N-([2R]-2-methylbutyl)amide, 2E-decen-4-ynoic acid N-isobutylamide, 2Z-decen-4-ynoic acid N-isobutylamide, 2E,6Z,8E,10E-dodecatetraenoic acid N-(2-methylpropyl)amide (α-sanshool), 2E,6Z,8E,10E-dodecatetraenoic acid N-(2-hydroxy-2-methylpropyl)amide (α-hydroxysanshool), 2E,6E,8E,10E-dodecatetraenoic acid N-(2-hydroxy-2-methylpropyl)amide (β-hydroxysanshool), 2E,4E,8Z,10E,12E-tetradecapentaenoic acid N-(2-hydroxy-2-methylpropyl)amide (γ-hydroxysanshool), 2E,4E,8E,10E,12E-tetradecapentaenoic acid N-(2-hydroxy-2-methylprypyl)amide (γ-hydroxyisosanshool), 2E,4E,8Z,10E,12E-tetradecapentaenoic acid N-(2-methyl-2-propenyl)amide (y-dehydrosanshool), 2E,4E,8Z,10E,12E-tetradecapentaenoic acid N-(2-methylpropyl)amide (y-sanshool), 2E,4E,8Z,11Z-tetradecatetraenoic acid N-(2-hydroxy-2-methylpropyl)amide (bungeanool), 2E,4E,8Z,11E-tetradecatetraenoic acid N-(2-hydroxy-2-methylpropyl)amide (isobungeanool), 2E,4E,8Z-tetradecatrienoic acid N-(2-hydroxy-2-methylpropyl)amide (dihydrobungeanool) and 2E,4E-tetradecadienoic acid N-(2-hydroxy-2-methylpropyl)amide (tetrahydrobungeanool);
(ii) is hesperetin of the formula (I) 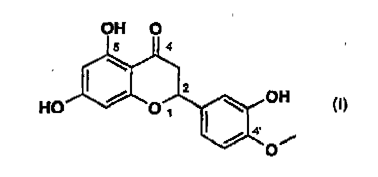 wherein the hesperetin of the formula (I) is present as the (2S)-enantiomer, (2R)-enantiomer or any desired mixture of the two enantiomers, and/or salts thereof;
and
(iii) is
- a 4-hydroxydihydrochalcone of the formula (II) 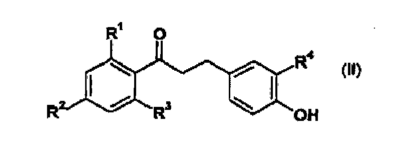 wherein
R¹, R², R³ and R⁴ independently of each other in each case denote H, OH or O-alkyl, with the proviso that at least one of the radicals R¹, R² or R³ denotes OH,
- a salt of such a 4-hydroxydihydrochalcone of the formula (II),
- a mixture comprising or consisting of two or more different 4-hydroxydihydrochalcones of the formula (II), wherein R¹, R², R³ and R⁴ in each case have the abovementioned meaning,
- a mixture comprising or consisting of salts of two or more different 4-hydroxydihydrochalcones of the formula (II), wherein R¹, R², R³ and R⁴ in each case have the abovementioned meaning,
or
- a mixture comprising or consisting of a 4-hydroxydihydrochalcone of the formula (II) or two or more different 4-hydroxydihydrochalcones of the formula (II), wherein R¹, R² , R³ and R⁴ in each case have the abovementioned meaning, and a salt of a 4-hydroxydihydrochalcone of the formula (II) or two or more salts of different 4-hydroxydihydrochalcones of the formula (II), wherein R¹, R² R³ and R⁴ in each case have the abovementioned meaning.

2. Aroma composition according to claim 1, wherein:
(i) consists of one or more alkamides chosen from the group consisting of:
2E,4E-decadienoic acid N-isobutylamide (pellitorine), 2E,4Z-decadienoic acid N-isobutylamide (cis-pellitorine), 2Z,4Z-decadienoic acid N-isobutylamide, 2Z,4E-decadienoic acid N-isobutylamide, 2E,4E-decadienoic acid N-piperide (achilleamide), 2E,6Z,8E-decatrienoic acid N-isobutylamide (spilanthol), 2E,6Z,8E-decatrienoic acid N-([2S]-2-methylbutyl)amide (homospilanthol), 2E,6Z,SE-decatrienoic acid N-([2R]-2-methylbutyl)amide, 2E,6Z,82,10E-dodecatetraenoic acid N-(2-methylpropyl) amide (α-sanshool), 2E,6Z,8E,10E-dodecatetraenoic acid N-(2-hydroxy-2-methylpropyl)amide (α-hydroxysanshool), 2E,4E,8Z,10E,12E-tetradecapentaenoic acid N-(2-methylpropyl)amide (y-sanshool) and 2E,4E,8Z,11Z-tetradecatetraenoic acid N-(2-hydroxy-2-methylpropyl)amide (bungeanool);
and/or
(ii) is hesperetin of the formula (I) 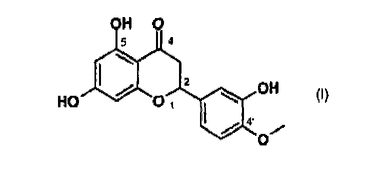 wherein the hesperetin of the formula (I) is present as the (2S)-enantiomer, (2R)-enantiomer or any desired mixture of the two enantiomers,
and/or
(iii) is a 4-hydroxydihydrochalcone of the formula (II), wherein in the formula (II) in each case
R¹ denotes OH
R² and R³ independently of each other denote H or OH,
and
R⁴ denotes H or methoxy (OCH₃).

3. Aroma composition according to one of the preceding claims, wherein:
(i) consists of one or more alkamides chosen from the group consisting of:
2E,4E-decadienoic acid N-isobutylamide (pellitorine), 2E,4Z-decadienoic acid N-isobutylamide (cis-pellitorine), 2Z,4Z-decadienoic acid N-isobutylamide, 2Z,4E-decadienoic acid N-isobutylamide, 2E,6Z,8E-decatrienoic acid N-isobutylamide (spilanthol), 2E,6Z,8E,10E-dodecatetraenoic acid N-(2-methylpropyl)amide (α-sanshool), 2E,4E,8Z,11Z-tetradecatetraenoic acid N-(2-hydroxy-2-methylpropyl)amide (bungeanool);
and/or
(ii) is hesperetin of the formula (I) 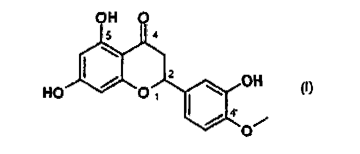 wherein the hesperetin of the formula (I) is present as the (2S)-enantiomer or any desired mixture of the two enantiomers,
and/or
(iii) consists of
- a 4-hydroxydihydrochalcone of the formula (II) chosen from the group consisting of:
3-(4-hydroxyphenyl)-1-(2-hydroxyphenyl)propan-1-one (2',4-dihydroxydihydrochalcone; compound 1),
3-(4-hydroxyphenyl)-1-(2,4-dihydroxyphenyl)propan-1-one (2',4,4'-trihydroxydihydrochalcone; davidigenin; compound 2),
3-(4-hydroxyphenyl)-1-(2,6-dihydroxyphenyl)propan-1-one (2',4,6'-trihydroxydihydrochalcone; compound 3),
3-(4-hydroxyphenyl)-1-(2,4,6-trihydroxyphenyl)propan-1-one (2',4,4',6'-tetrahydroxydihydrochalcone; phloretin; compound 4),
3-(4-hydroxy-3-methoxyphenyl)-1-(2-hydroxyphenyl)propan-1-one (2',4-dihydroxy-3-methoxydihydrochalcone; compound 5),
3-(4-hydroxy-3-methoxyphenyl)-1-(2,4-dihydroxyphenyl)propan-1-one (2',4,4'-trihydroxy-3-methoxydihydrochalcone; compound 6),
3-(4-hydroxy-3-methoxyphenyl)-1-(2,6-dihydroxyphenyl)propan-1-one (2',4,6'-trihydroxy-3-methoxydihydrochalcone; compound 7),
3-(4-hydroxy-3-methoxyphenyl)-1-(2,4,6-trihydroxyphenyl)propan-1-one (2',4,4',6'-tetrahydroxy-3-methoxydihydrochalcone; compound 8) and
3-(3,4-dihydroxyphenyl)-1-(2,4,6-trihydroxyphenyl)propan-1-one (2',3,4,4',6'-tetrahydroxydihydrochalcone; compound 9),
- a salt of such a 4-hydroxydihydrochalcone of the formula (II),
- a mixture comprising or consisting of two or more 4-hydroxydihydrochalcones of the formula (II) chosen from the said group,
- a mixture comprising or consisting of salts of two or more 4-hydroxydihydrochalcones of the formula (II) chosen from the said group,
or
- a mixture comprising or consisting of
one of the 4-hydroxydihydrochalcones of the formula (II) chosen from the said group or two or more 4-hydroxydihydrochalcones of the formula (II) chosen from the said group and
a salt of a 4-hydroxydihydrochalcone of the formula (II) chosen from the said group or two or more salts of different 4-hydroxydihydrochalcones of the formula (II) chosen from the said group.

4. Aroma composition according to one of the preceding claims, wherein:
(i) consists of one or more alkamides chosen from the group consisting of:
2E,4E-decadienoic acid N-isobutylamide (pellitorine), 2E,6Z,8E-decatrienoic acid N-isobutylamide (spilanthol), 2E,6Z,8E,10E-dodecatetraenoic acid N-(2-methylpropyl)amide (α-sanshool) and 2E,4E,8Z,11Z-tetradecatetraenoic acid N-(2-hydroxy-2-methylpropyl)amide (bungeanool);
and/or
(ii) is hesperetin of the formula (I) 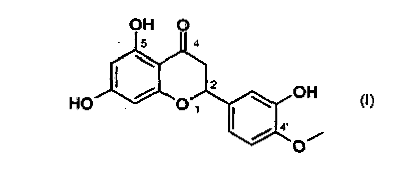 wherein the hesperetin of the formula (I) is present as the (2S)-enantiomer or any desired mixture of the two enantiomers,
and/or
(iii) consists of or comprises phloretin (compound 4).

5. Aroma composition according to one of the preceding claims, wherein
- the weight ratio of the total amount of substances (i) to the total amount of substances (ii) and (iii) is in a range from 1:1,000,000 to 1:1, preferably in a range of from 1:10,000 to 1:10, particularly preferably in a range of from 1:2,000 to 1:50
and/or
- the weight ratio of the total amounts of substances (ii) to substances (iii) is in the range from 1:10 to 10:1, particularly preferably in the range of from 5:1 to 1:5 and especially preferably in the range of from 7:3 to 3:7.

6. Use of an aroma composition according to one of the preceding claims
- to enhance the sweet flavour of a sweet-tasting substance or the sweet odour impression of an aroma substance which gives rise to a sweet odour impression,
or
- to enhance the initial sweetness of a sweet-tasting substance or of an aroma substance which gives rise to a sweet odour impression.

7. Preparation from the group consisting of preparations serving for nutrition, for oral care or for pleasure, semi-finished products, odoriferous, aroma or flavour substance compositions or seasoning mixtures comprising the following components:
(a) an aroma composition according to one of the preceding claims 1 to 5
and additionally
(b) one or more sweet-tasting substances and/or
(c) one or more aroma substances which give rise to a sweet odour impression,
wherein the total amount of component (a) in the preparation is sufficient to sensorially enhance the sweet flavour impression of the sweet-tasting substance(s) (b) or the sweet odour impression of the aroma substance(s) (c) which give rise to a sweet odour impression.

8. Preparation according to claim 7, comprising as component (b) one or more sugars, wherein the total amount of component (a) in the preparation is sufficient to impart, in comparison with a preparation or semi-finished product which, having an otherwise identical composition, comprises no component (a) but at least 1.05 times the amount of sugar, the same or an enhanced initial sweetness.

9. Preparation according to one of claims 7 or 8, comprising
(b) one or more further sweet-tasting substances, wherein the further sweet-tasting substance(s) are chosen from the group consisting of:
- one or more carbohydrates chosen from the group consisting of sucrose, trehalose, lactose, maltose, melizitose, melibiose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde, maltodextrin and plant preparations containing one or more of the carbohydrates mentioned,
- one or more sugar alcohols chosen from the group consisting of glycerol, erythritol, threitol, arabitol, ribitol, xylitol, sorbitol, mannitol, maltitol, isomaltitol, dulcitol and lactitol,
- one or more proteins and/or amino acids from the group consisting of miraculin, monellin, thaumatin, curculin, brazzein, glycine, D-leucine, D-threonine, D-asparagine, D-phenylalanine, D-tryptophan, L-proline,
- one or more sweeteners from the group consisting of MAGAP, sodium cyclamate, acesulfame K, neohesperidin dihydrochalcone, saccharin sodium salt, aspartame, superaspartame, neotame, alitame, sucralose, stevioside, rebaudioside, lugduname, carrelame, sucrononate, sucrooctate, monatin and phyllodulcin,
and mixtures thereof
and/or
(c) one or more aroma substances which give rise to a sweet odour impression, wherein the further aroma substance(s) which give rise to a sweet odour impression are chosen from the group consisting of:
vanillin, ethylvanillin, ethylvanillin isobutyrate (= 3-ethoxy-4-isobutyryloxybenzaldehyde), Furaneol^{®} (2,5-dimethyl-4-hydroxy-3(2H)-furanone) and derivatives (e.g. homofuraneol, 2-ethyl-4-hydroxy-5-methyl-3(2H)-furanone), homofuronol (2-ethyl-5-methyl-4-hydroxy-3(2H)-furanone and 5-ethyl-2-methyl-4-hydroxy-3(2H)-furanone), maltol and derivatives (e.g. ethylmaltol), coumarin and derivatives, gamma-lactones (e.g. gamma-undecalactone, gamma-nonalactone), delta-lactones (e.g. 4-methyl-delta-lactone, massoilactone, delta-decalactone, tuberolactone), methyl sorbate, divanillin, 4-hydroxy-2(or 5)-ethyl-5(or 2)-methyl-3(2H)furanone, 2-hydroxy-3-methyl-2-cyclopentenones, 3-hydroxy-4,5-dimethyl-2(5H)-furanone, fruit esters and fruit lactones (e.g. acetic acid n-butyl ester, acetic acid isoamyl ester, propionic acid ethyl ester, butyric acid ethyl ester, butyric acid n-butyl ester, butyric acid isoamyl ester, 3-methyl-butyric acid ethyl ester, n-hexanoic acid ethyl ester, n-hexanoic acid allyl ester, n-hexanoic acid n-butyl ester, n-octanoic acid ethyl ester, ethyl 3-methyl-3-phenyl glycidate, ethyl 2-trans-4-cis-decadienoate), 4-(p-hydroxyphenyl)-2-butanone, 1,1-dimethoxy-2,2,5-trimethyl-4-hexane, 2,6-dimethyl-5-hepten-1-al and phenylacetaldehyde.

10. Preparation according to one of claims 7 to 9 serving for nutrition, for oral care or for pleasure, comprising a total amount of an aroma composition according to one of claims 1 to 4, based on the total weight of the preparation, in the range of from 0.1 to 150 ppm, preferably in the range of from 1 to 50 ppm, particularly preferably in the range of from 10 to 50 ppm.

11. Preparation according to one of claims 7 to 10 serving for nutrition, for oral care or for pleasure, wherein the total amount
- of substances (i) is in the range from 0.005 to 5 ppm, preferably 0.02 to 2 ppm, particularly preferably 0.05 to 0.5 ppm,
- of substances (ii) is in the range from 0.5 to 500 ppm, preferably 10 to 200 ppm, particularly preferably 20 to 100 ppm
and/or
- of substances (iii) is in the range from 0.5 to 500 ppm, preferably 5 to 100 ppm, particularly preferably 10 to 40 ppm,
wherein the total amount of all components (i), (ii) and (iii) is in the range from 0.5 to 500 ppm, preferably in the range of from 5 to 200 ppm, particularly preferably in the range of from 10 to 100 ppm.

12. Preparation according to one of claims 7 to 11 serving for nutrition, for oral care or for pleasure, wherein the preparation is chosen from the group consisting of:
(A) confectionery,
(B) alcoholic or non-alcoholic beverages or instant beverages,
(C) cereal products and/or nut products,
(D) dairy products,
(E) products made from soya protein or other soya bean fractions,
(F) fruit and/or vegetable preparations,
(G) fat- and oil-based products or emulsions thereof, and
(H) oral care products.

13. Preparation according to one of claims 7 to 12, chosen from the group consisting of semi-finished products, odoriferous, aroma or flavour substance compositions or seasoning mixtures, comprising a total amount of an aroma composition according to one of claims 1 to 5, based on the total weight of the preparation, in the range of from 0.0001 wt.% to 95 wt.%, preferably 0.001 wt.% to 80 wt.%, particularly preferably 0.001 wt.% to 50 wt.%.

14. Method for enhancing the sweet taste or the initial sweetness of a sweet-tasting substance or the sweet odour impression or the initial sweetness of an aroma substance which gives rise to a sweet odour impression, with the following step:
- mixing of one or more sweet-tasting substances (component (b)) or one or more aroma substances which give rise to a sweet odour impression (component (c)), with a total amount of an aroma composition according to the invention (component (a)) according to one of claims 1 to 5, wherein the total amount of aroma composition according to the invention (component (a)) in the preparation is sufficient to sensorially enhance the sweet flavour impression of the sweet-tasting substance(s) (b) or the sweet odour impression of the aroma substance(s) (c) which give rise to a sweet odour impression, and in particular the initial sweetness.

15. Use of one or more alkamides (i) chosen from the group consisting of:
2E,4E-decadienoic acid N-isobutylamide (pellitorine), 2E,4Z-decadienoic acid N-isobutylamide (cis-pellitorine), 2Z,4Z-decadienoic acid N-isobutylamide, 2Z,4E-decadienoic acid N-isobutylamide, 2E,4E-decadienoic acid N-([2S]-2-methylbutyl)amide, 2E,4E-decadienoic acid N-([2S]-2-methylbutyl)amide, 2E,4E-decadienoic acid N-([2R]-2-methylbutyl)amide, 2E,4Z-decadienoic acid N-(2-methylbutyl)amide, 2E,4E-decadienoic acid N-piperide (achilleamide), 2E,4E-decadienoic acid N-piperide (sarmentine), 2E-decenoic acid N-isobutylamide, 3E-decenoic acid N-isobutylamide, 3E-nonenoic acid N-isobutylamide, 2E,6Z,8E-decatrienoic acid N-isobutylamide (spilanthol), 2E,6Z,8E-decatrienoic acid N-([2S]-2-methylbutyl)amide (homospilanthol), 2E,6Z,8E-decatrienoic acid N-([2R]-2-methylbutyl)amide, 2E-decen-4-ynoic acid N-isobutylamide, 2Z-decen-4-ynoic acid N-isobutylamide, 2E,6Z,8E,10E-dodecatetraenoic acid N-(2-methylpropyl)amide (α-sanshool), 2E,6Z,8E,10E-dodecatetraenoic acid N-(2-hydroxy-2-methylpropyl)amide (α-hydroxysanshool), 2E,6E,8E,10E-dodecatetraenoic acid N-(2-hydroxy-2-methylpropyl)amide (β-hydroxysanshool), 2E,4E,8Z,10E,12E-tetradecapentaenoic acid N-(2-hydroxy-2-methylpropyl)amide (y-hydroxysanshool), 2E,4E,8E,10E,12E-tetradecapentaenoic acid N-(2-hydroxy-2-methylpropyl)amide (γ-hydroxyisosanshool), 2E,4E,8Z,10E,12E-tetradecapentaenoic acid N-(2-methyl-2-propenyl)amide (γ-dehydrosanshool), 2E,4E,8Z,10E,12E-tetradecapentaenoic acid N-(2-methylpropyl)amide (y-sanshool), 2E,4E,8Z,11Z-tetradecatetraenoic acid N-(2-hydroxy-2-methylpropyl)amide (bungeanool), 2E,4E,8Z,11E-tetradecatetraenoic acid N-(2-hydroxy-2-methylpropyl)amide (isobungeanool), 2E,4E,8Z-tetradecatrienoic acid N-(2-hydroxy-2-methylpropyl)amide (dihydrobungeanool) and 2E,4E-tetradecadienoic acid N-(2-hydroxy-2-methylpropyl)amide (tetrahydrobungeanool);
to enhance the initial sweetness of a preparation comprising
(ii) hesperetin of the formula (I) 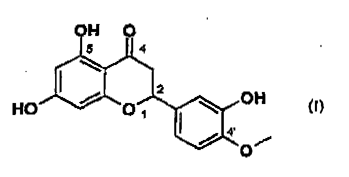 wherein the hesperetin of the formula (I) is present as the (2S)-enantiomer, (2R)-enantiomer or any desired mixture of the two enantiomers, and/or salts thereof;
and/or
(iii)
a 4-hydroxydihydrochalcone of the formula (II) 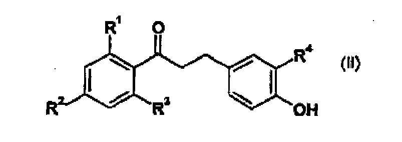
wherein
R¹, R², R³ and R⁴ independently of each other in each case denote H, OH or O-alkyl, with the proviso that at least one of the radicals R¹, R² or R³ denotes OH,
- a salt of such a 4-hydroxydihydrochalcone of the formula (II),
- a mixture comprising or consisting of two or more different 4-hydroxydihydrochalcones of the formula (II), wherein R¹, R², R³ and R⁴ in each case have the abovementioned meaning,
- a mixture comprising or consisting of salts of two or more different 4-hydroxydihydrochalcones of the formula (II), wherein R¹, R², R³ and R⁴ in each case have the abovementioned meaning, or
- a mixture comprising or consisting of a 4-hydroxydihydrochalcone of the formula (II) or two or more different 4-hydroxydihydrochalcones of the formula (II), wherein R¹, R², R³ and R⁴ in each case have the abovementioned meaning, and
a salt of a 4-hydroxydihydrochalcone of the formula (II) or two or more salts of different 4-hydroxydihydrochalcones of the formula (II), wherein R¹, R², R³ and R⁴ in each case have the abovementioned meaning,13
and comprising
(b) one or more sweet-tasting substances and/or
(c) one or more aroma substances which give rise to a sweet odour impression.

## Revendications

1. Composition aromatique, composée de ou contenant une substance (i) ainsi que soit:
- une substance (ii), soit
- une substance (iii), soit
- un mélange des substances (ii) et (iii), dans laquelle:
(i) est composé d'un ou de plusieurs alcamides choisis dans le groupe constitué de:
acide 2E,4E-décadiénique-N-isobutylamide (pellitorine), acide 2E,4Z-décadiénique-N-isobutylamide (cis-pellitorine), acide 2Z,4Z-décadiénique-N-isobutylamide, acide 2Z,4E-décadiénique-N-isobutylamide, acide 2E,4E-décadiénique-N-([2S]-2-méthylbutyl)amide, acide 2E,4E-décadiénique-N-([2S]-2-méthylbutyl)amide, acide 2E,4E-décadiénique-N-([2R]-2-méthylbutyl)amide, acide 2E,4Z-décadiénique-N-(2-méthylbutyl)amide, acide 2E,4E-décadiénique-N-pipéride (achilleamide), acide 2E,4E-décadiénique-N-pipéride (sarmentine), acide 2E-decénique-N-isobutylamide, acide 3E-décénique-N-isobutylamide, acide 3E-nonénique-N-isobutylamide, acide 2E,6Z,8E-décatriénique-N-isobutylamide (spilanthol), acide 2E,6Z,8E-décatriénique-N-([2S]-2-méthylbutyl)amide (homospilanthol), acide 2E,6Z,8E-décatriénique-N-([2R]-2-méthylbutyl)amide, acide 2E-décén-4-ique-N-isobutylamide, acide 2Z-décén-4-ique-N-isobutylamide, acide 2E,6Z,8E,10E-dodécatétraénique-N-(2-méthylpropyl)amide (a-sanshool), acide 2E,6Z,8E,10E-dodécatétraénique-N-(2-hydroxy-2-méthylpropyl)amide (α-hydroxysanshool), acide 2E,6E,8E,10E-dodécatétraénique-N-(2-hydroxy-2-méthylpropyl)amide (β-hydroxysanshool), acide 2E,4E,8Z,10E,12E-tétradécapentaénique-N-(2-hydroxy-2-méthylpropyl)amide (γ-hydroxysanshool), acide 2E,4E,8E,10E,12E-tétradécapentaénique-N-(2-hydroxy-2-méthylpropyl)amide (γ-hydroxyisosanshool), acide 2E,4E,8Z,10E,12E-tétradécapentaénique-N-(2-méthyl-2-propényl)amide (γ-déshydrosanshool), acide 2E,4E,8Z,10E,12E-tétradécapentaénique-N-(2-méthylpropyl)amide (γ-sanshool), acide 2E,4E,8Z,11Z-tétradécatétraénique-N-(2-hydroxy-2-méthylpropyl)amide (bungeanool), acide 2E,4E,8Z,11E-tétradécatétraénique-N-(2-hydroxy-2-méthylpropyl)amide (isobungeanool), acide 2E,4E,8Z-tétradécatriénique-N-(2-hydroxy-2-méthylpropyl)amide (dihydrobungeanool) et acide 2E,4E-tétradécadiénique-N-(2-hydroxy-2-méthylpropyl)amide (tétrahydrobungeanool);
(ii) est l'hespérétine de la formule (I): 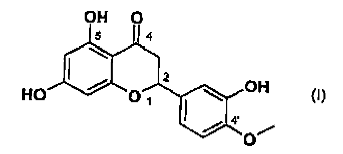 dans laquelle l'hespérétine de la formule (I) existe sous la forme de l'énantiomère (2S), de l'énantiomère (2R) ou d'un mélange quelconque des deux énantiomères, et/ou des sels de celle-ci,
et
(iii) est:
- une 4-hydroxydihydrochalcone de la formule (II): 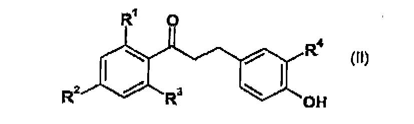 dans laquelle:
R¹, R², R³ et R⁴ signifient indépendamment les uns des autres respectivement H, OH ou un O-alkyle, sous réserve que au moins une des fractions reste de R¹, R² ou R³ signifie OH,
- un sel d'une 4-hydroxydihydrochalcone de la formule (II),
- un mélange comprenant ou composé de deux ou plus 4-hydroxydihydrochalcones différentes de la formule (II), dans laquelle R¹, R², R³ et R⁴ ont respectivement la signification indiquée ci-dessus,
- un mélange comprenant ou composé de sels de deux ou plus 4-hydroxydihydrochalcones différentes de la formule (II), dans laquelle R¹, R², R³ et R⁴ ont respectivement la signification indiquée ci-dessus,
ou
- un mélange comprenant ou composé d'une 4-hydroxydihydrochalcone de la formule (II) ou de deux ou plus 4-hydroxydihydrochalcones différentes de la formule (II), dans laquelle R¹, R², R³ et R⁴ ont respectivement la signification indiquée ci-dessus, et d'un sel d'une 4-hydroxydihydrochalcone de la formule (II) ou de deux sels ou plus de 4-hydroxydihydrochalcones différentes de la formule (II), dans laquelle R¹, R², R³ et R⁴ ont respectivement la signification indiquée ci-dessus.

2. Composition aromatique selon la revendication 1, dans laquelle:
(i) est composé d'un ou de plusieurs alcamides choisis dans le groupe constitué de:
acide 2E,4E-décadiénique-N-isobutylamide (pellitorine), acide 2E,4Z-décadiénique-N-isobutylamide (cis-pellitorine), acide 2Z,4Z-décadiénique-N-isobutylamide, acide 2Z,4E-décadiénique-N-isobutylamide, acide 2E,4E-décadiénique-N-pipéride (achilleamide), acide 2E,6Z,8E-décatriénique-N-isobutylamide (spilanthol), acide 2E,6Z,SE-décatriénique-N-([2S]-2-méthylbutyl)amide (homospilanthol), acide 2E,6Z,8E-décatriénique-N-([2R]-2-méthylbutyl)amide, acide 2E,6Z,8E,10E-dodécatétraénique-N-(2-méthylpropyl)amide (α-sanshool), acide 2E,6Z,8E,10E-dodécatétraénique-N-(2-hydroxy-2-méthylpropyl)amide (α-hydroxysanshool), acide 2E,4E,8Z,10E,12E-tétradécapentaénique-N-(2-méthylpropyl)amide (γ-sanshool) et acide 2E,4E,8Z,11Z-tétradécatétraénique-N-(2-hydroxy-2-méthylpropyl)amide (bungeanool);
et/ou
(ii) est l'hespérétine de la formule (I): 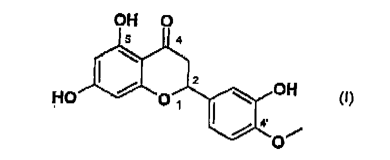 dans laquelle l'hespérétine de la formule (I) existe sous la forme de l'énantiomère (2S), de l'énantiomère (2R) ou d'un mélange quelconque des deux énantiomères,
et/ou
(iii) est une 4-hydroxydihydrochalcone de la formule (II), où dans la formule (II) respectivement:
R¹ signifie OH,
R²'et R³ signifient, indépendamment l'un de l'autre, H ou OH,
et
R⁴ signifie H ou un méthoxy (OCH₃).

3. Composition aromatique selon l'une quelconque des revendications précédentes, dans laquelle:
(i) est composé d'un ou de plusieurs alcamides choisis dans le groupe constitué de:
acide 2E,4E-décadiénique-N-isobutylamide (pellitorine), acide 2E,4Z-décadiénique-N-isobutylamide (cis-pellitorine), acide 2Z,4Z-décadiénique-N-isobutylamide, acide 2Z,4E-décadiénique-N-isobutylamide, acide 2E,6Z,8E-décatriénique-N-isobutylamide (spilanthol), acide 2E,6Z,8E,l0E-dodécatétraénique-N-(2-méthylpropyl)amide (α-sanshool), acide 2E,4E,8Z,11Z-tétradécatétraénique-N-(2-hydroxy-2-méthylpropyl)amide (bungeanool);
et/ou
(ii) est l'hespérétine de la formule (I): 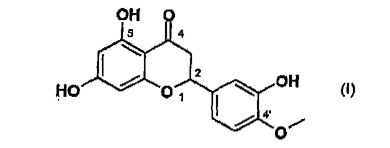 dans laquelle l'hespérétine de la formule (I) existe sous la forme de l'énantiomère (2S) ou d'un mélange quelconque des deux énantiomères,
et/ou
(iii) est composé de:
- une 4-hydroxydihydrochalcone de la formule (II) choisie dans le groupe constitué de:
3-(4-hydroxyphényl)-1-(2-hydroxyphényl)propan-1-one (2',4-dihydroxydihydrochalcone; composé 1), 3-(4-hydroxyphényl)-1-(2,4-dihydroxyphényl)propan-1-one (2',4,4'-trihydroxydihydrochalcone; davidigénine; composé 2), 3-(4-hydroxyphényl)-1-(2,6-dihydroxyphényl)propan-1-one (2',4,6'-trihydroxydihydrochalcone; composé 3), 3-(4-hydroxyphényl)-1-(2,4,6-trihydroxyphényl)propan-1-one (2',4,4',6'-tétrahydroxydihydrochalcone; phlorétine; composé 4), 3-(4-hydroxy-3-méthoxyphényl)-1-(2-hydroxyphényl)propan-1-one (2',4-dihydroxy-3-methoxydihydrochalcone; composé 5), 3-(4-hydroxy-3-méthoxyphényl)-1-(2,4-dihydroxyphényl)propan-1-one (2',4,4'-trihydroxy-3-méthoxydihydrochalcone; composé 6), 3-(4-hydroxy-3-méthoxyphényl)-1-(2,6-dihydroxyphényl)propan-1-one (2',4,6'-trihydroxy-3-méthoxydihydrochalcone; composé 7), 3-(4-hydroxy-3-méthoxyphényl)-1-(2,4,6-trihydroxyphényl)propan-1-one (2',4,4',6'-tétrahydroxy-3-méthoxydihydrochalcone; composé 8), et 3-(3,4-dihydroxyphényl)-1-(2,4,6-trihydroxyphényl)propan-1-one (2',3,4,4',6'-tétrahydroxydihydrochalcone; composé 9),
- un sel de 4-hydroxydihydrochalcones de la formule (II),
- un mélange comprenant ou composé de deux ou plus 4-hydroxydihydrochalcones de la formule (II) choisies dans le groupe susmentionné,
- un mélange comprenant ou composé de sels de deux ou plus 4-hydroxydihydrochalcones de la formule (II) choisies dans le groupe susmentionné,
ou
- un mélange comprenant ou composé de:
une des 4-hydroxydihydrochalcones de la formule (II) choisies dans le groupe susmentionné ou deux ou plus 4-hydroxydihydrochalcones de la formule (II) choisies dans le groupe susmentionné, et
un sel d'une 4-hydroxydihydrochalcone de la formule (II) choisie dans le groupe susmentionné ou deux sels ou plus de 4-hydroxydihydrochalcones différentes de la formule (II) choisies dans le groupe susmentionné.

4. Composition aromatique selon l'une quelconque des revendications précédentes, dans laquelle.
(i) est composé d'un ou de plusieurs alcamides choisis dans le groupe constitué de:
acide 2E,4E-décadiénique-N-isobutylamide (pellitorine), acide 2E,6Z,8E-décatriénique-N-isobutylamide (spilanthol), acide 2E,62,8E,10E-dodécatétraénique-N-(2-méthylpropyl)amide (α-sanshool) et acide 2E,4E,8Z,11Z-tétradécatétraénique-N-(2-hydroxy-2-méthylpropyl)amide (bungeanool);
et/ou
(ii) est l'hespérétine de la formule (I); 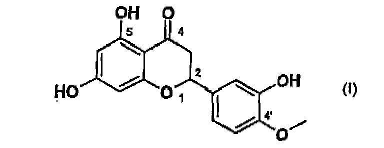 dans laquelle l'hespérétine de la formule (I) existe sous la forme de l'énantiomère (2S) ou d'un mélange quelconque des deux énantiomères,
et/ou
(iii) est composé de ou comprend la phlorétine (composé 4).

5. Composition aromatique selon l'une quelconque des revendications précédentes, dans laquelle:
- le rapport en poids de la quantité totale des substances (i) sur la quantité totale des substances (ii) et (iii) se trouve dans un intervalle de 1:1.000.000 à 1:1, de préférence dans un intervalle de 1:10.000 à 1:10, plus préférablement dans un intervalle de 1:2.000 à 1:50,
et/ou
- le rapport en poids des quantités totales des substances (ii) sur les substances (iii) se trouve dans un intervalle de 1:10 à 10:1, plus préférablement dans un intervalle de 5:1 à 1:5 et le plus préférablement dans un intervalle de 7:3 à 3:7.

6. Utilisation d'une composition aromatique selon l'une quelconque des revendications précédentes:
- pour renforcer le goût sucré d'une matière donnant un goût sucré ou l'impression d'odeur sucrée d'une matière aromatisante, qui provoque une impression d'odeur sucrée, ou
- pour renforcer le début de sucré d'une matière donnant un goût sucré ou d'une matière aromatisante, qui provoque une impression d'odeur sucrée.

7. Préparation issue du groupe constitué de produits semi-finis, de compositions de matières odorantes, aromatisantes ou donnant du goût ou de mélanges de parfums destinés à l'alimentation, à l'hygiène buccale ou à la consommation, comprenant les composants suivants:
(a) une composition aromatique selon l'une quelconque des revendications 1 à 5 précédentes,
ainsi que en outre
(b) une ou plusieurs matières donnant un goût sucré,
et/ou
(c) une ou plusieurs matières aromatisantes, qui provoquent une impression d'odeur sucrée,
dans laquelle la quantité totale du composant (a) dans la préparation est suffisante pour renforcer de façon sensorielle l'impression de goût sucré de la ou des matière(s) donnant un goût sucré (b) ou l'impression d'odeur sucrée de la ou des matières aromatisantes (c), qui provoquent une impression d'odeur sucrée.

8. Préparation selon la revendication 7, comprenant en tant que composant (b) un ou plusieurs sucres, où la quantité totale du composant (a) dans la préparation est suffisante, en comparaison avec une préparation ou un produit semi-fini, qui est une composition identique mis à part le fait de ne pas comprendre de composant (a) mais de comprendre au moins 1,05 fois la quantité de sucre, pour procurer un début de sucré identique ou renforcé.

9. Préparation selon l'une quelconque des revendications 7 ou 8, comprenant:
(b) une ou plusieurs matières donnant un goût sucré supplémentaires, ou la ou les matières donnant un goût sucré supplémentaires sont choisies dans le groupe constitué de:
- un ou plusieurs hydrates de carbone choisis dans le groupe constitué de saccharose, tréhalose, lactose, maltose, mélézitose, mélibiose, raffinose, palatinose, lactulose, D-fructose, D-glucose, D-galactose, L-rhamnose, D-sorbose, D-mannose, D-tagatose, D-arabinose, L-arabinose, D-ribose, D-glycéraldéhyde, maltodextrine et des préparations végétales contenant un ou plusieurs des hydrates de carbone nommés,
- un ou plusieurs alcools de sucre choisis dans le groupe constitué de glycérine, érythritol, thréitol, arabitol, ribitol, xylitol, sorbitol, mannitol, malitol, isomaltit, dulcitol et lactilol,
- une ou plusieurs protéines et/ou un ou plusieurs acides aminés provenant du groupe constitué de miraculine, monelline, thaumatine, curculine, brazzéine, glycine, D-leucine, D-thréonine, D-asparagine, D-phénylalanine, D-tryptophane, L-proline,
- un ou plusieurs édulcorants provenant du groupe constitué de magape, cyclamate de sodium, acésulfame K, néohespéridinedihydrochalcone, saccharine-sel de sodium, aspartame, superaspartame, néotame, alitame, sucralose, stévioside, rébaudioside, lugduname, carrélame, sucrononate, sucrooctate, monatine et phyllodulcine,
et leurs mélanges
et/ou
(c) une ou plusieurs matières aromatisantes, qui provoquent une impression d'odeur sucrée, où la ou les matières aromatisantes supplémentaires, qui provoquent une impression d'odeur sucrée, sont choisies dans le groupe constitué de:
vanilline, éthylvanilline, isobutyrate d'éthylvanilline (= 3-éthoxy-4-isobutyryloxybenzaldéhyde), furanéol (2,5-diméthyl-4-hydroxy-3(2H)-furanone) et dérivés (par ex. homofuranéol, 2-éthyl-4-hydroxy-5-méthyl-3(2H)-furanone), homofuronol (2-éthyl-5-méthyl-4-hydroxy-3(2H)-furanone et 5-éthyl-2-méthyl-4-hydroxy-3(2H)-furanone), maltol et dérivés (par ex. éthylmaltol), cumarine et dérivés, gamma-lactone (par ex. gamma-undécalactone, gamma-nonalactone), delta-lactone (par ex. 4-méthyldeltalactone, massoilactone, deltadécalactone, tubérolactone), méthylsorbate, divanilline, 4-hydroxy-2(ou 5)-éthyl-5(ou 2)méthyl-3(2H)-furanone, 2-hydroxy-3-méthyl-2-cyclopenténone, 3-hydroxy-4,5-diméthyl-2(5H)-furanone, ester de fruit et lactone de fruit (par ex. n-butylester d'acide acétique, isoamylester d'acide acétique, éthylester d'acide propionique, éthylester d'acide butyrique, n-butylester d'acide butyrique, isoamylester d'acide butyrique, éthylester d'acide 3-méthylbutyrique, éthylester d'acide n-hexanoïque, allylester d'acide n-hexanoïque, n-butylester d'acide n-hexanoïque, éthylester d'acide n-octanoïque, éthyl-3-méthyl-3-phénylglycidate, éthyl-2-trans-4-cis-décadiénoate), 4-(p-hydroxyphényl)-2-butanone, 1, 1-diméthoxy-2,2,5-triméthyl-4-hexane, 2,6-diméthyl-5-heptén-1-al et phénylacétaldéhyde.

10. Préparation destinée à l'alimentation, à l'hygiène buccale ou à la consommation selon l'une quelconque des revendications 7 à 9, comprenant une quantité totale dans l'intervalle de 0,1 à 150 ppm, de préférence dans l'intervalle de 1 à 50 ppm, plus préférablement dans l'intervalle de 10 à 50 ppm, d'une composition aromatique selon l'une quelconque des revendications 1 à 4, par rapport au poids total de la préparation.

11. Préparation destinée à l'alimentation, à l'hygiène buccale ou à la consommation selon l'une quelconque des revendications 7 à 10, dans laquelle la quantité totale,
- des substances (i) se trouve dans l'intervalle de 0,005 à 5 ppm, de préférence de 0,02 à 2 ppm, plus préférablement de 0,05 à 0,5 ppm,
des substances (ii) se trouve dans l'intervalle de 0,5 à 500 ppm, de préférence de 10 à 200 ppm, plus préférablement de 20 à 100 ppm,
et/ou
- des substances (iii) se trouve dans l'intervalle de 0,5 à 500 ppm, de préférence de 5 à 100 ppm, plus préférablement de 10 à 40 ppm,
dans laquelle la quantité totale des composants (i), (ii) et (iii) tous ensemble se trouve dans l'intervalle de 0,5 à 500 ppm, de préférence dans l'intervalle de 5 à 200 ppm, plus préférablement dans l'intervalle de 10 à 100 ppm.

12. Préparation servant à l'alimentation, à l'hygiène buccale ou à la consommation selon l'une quelconque des revendications 7 à 11, où la préparation est choisie dans le groupe constitué de:
(A) sucreries,
(B) boissons alcoolisées ou non alcoolisées ou boissons instantanées,
(C) produits à base de céréales et/ou produits à base de noisettes,
(D) produits laitiers,
(E) produits à base de protéine de soja ou d'autres fractions de graines de soja,
(F) préparations de fruits et/ou de légumes,
(G) produits à base de graisse et d'huile ou émulsions de ceux-ci,
(H) produits d'hygiène buccale.

13. Préparation selon l'une quelconque des revendications 7 à 12, choisie dans le groupe constitué de produits semi-finis, de compositions de matières odorantes, aromatisantes ou donnant du goût ou de mélanges de condiments, comprenant une quantité totale dans l'intervalle de 0,0001% en poids à 95% en poids, de préférence de 0,001% en poids à 80% en poids, plus préférablement de 0,001% en poids à 50% en poids, d'une composition aromatique selon l'une quelconque des revendications 1 à 5, par rapport au poids total de la préparation.

14. Procédé pour renforcer le goût sucré ou le début de sucré d'une matière donnant un goût sucré ou l'impression d'odeur sucrée ou le début de sucré d'une matière aromatisante, qui provoque une impression d'odeur sucrée, avec l'étape suivante:
- mélanger une ou plusieurs matières donnant un goût sucré (composant (b)) ou une ou plusieurs matières aromatisantes, qui provoque(nt) une impression d'odeur sucrée (composant (c)), avec une quantité totale d'une composition aromatique conforme à l'invention (composant (a)) selon l'une quelconque des revendications 1 à 5, où la quantité totale d'une composition aromatique conforme à l'invention (composant (a)) dans la préparation est suffisante pour renforcer de façon sensorielle l'impression de goût sucré de la ou des matières donnant un goût sucré (b) ou l'impression d'odeur sucrée de la ou des matières aromatisantes (c), qui provoquent une impression d'odeur sucrée, et particulièrement le début de sucré.

15. Utilisation d'un ou de plusieurs alcamides (i) choisis dans le groupe constitué de:
acide 2E,4E-décadiénique-N-isobutylamide (pellitorine), acide 2E,4Z-décadiénique-N-isobutylamide (cis-pellitorine), acide 2Z,4Z-décadiénique-N-isobutylamide, acide 2Z,4E-décadiénique-N-isobutylamide, acide 2E,4E-décadiénique-N-([2S]-2-méthylbutyl)amide, acide 2E,4E-décadiénique-N-([2S]-2-méthylbutyl)amide, acide 2E,4E-décadiénique-N-([2R]-2-méthylbutyl)amide, acide 2E,4Z-décadiénique-N-(2-méthylbutyl)amide, acide 2E,4E-décadiénique-N-pipéride (achilleamide), acide 2E,4E-décadiénique-N-pipéride (sarmentine), acide 2E-décénique-N-isobutylamide, acide 3E-décénique-N-isobutylamide, acide 3E-nonénique-N-isobutylamide, acide 2E,6Z,8E-décatriénique-N-isobutylamide (spilanthol), acide 2E,6Z,8E-décatriénique-N-([2S]-2-méthylbutyl)amide (homospilanthol), acide 2E,6Z,8E-décatriénique-N-([2R]-2-méthylbutyl)amide, acide 2E-décén-4-ique-N-isobutylamide, acide 2Z-décén-4-ique-N-isobutylamide, acide 2E,6Z,8E,10E-dodécatétraénique-N-(2-méthylpropyl)amide (a-sanshool), acide 2E,62,8E,10E-dodécatétraénique-N-(2-hydroxy-2-méthylpropyl)amide (α-hydroxysanshool), acide 2E,6B,8E,10E-dodécatétraénique-N-(2-hydroxy-2-méthylpropyl)amide (β-hydroxysanshool), acide 2E,4E,8Z,10E,12E-tétradécapentaénique-N-(2-hydroxy-2-méthylpropyl)amide (γ-hydroxysanshool), acide 2E,4E,8E,10E,12E-tétradécapentaénique-N-(2-hydroxy-2-méthylpropyl)amide (γ-hydroxyisosanshool), acide 2E,4E,8Z,10E,12E-tétradécapentaénique-N-(2-méthyl-2-propényl)amide (γ-déshydrosanshool), acide 2E,4E,8Z,10E,12E-tétradécapentaénique-N-(2-méthylpropyl)amide (γ-sanshool), acide 2E,4E,8Z,11Z-tétradécatétraénique-N-(2-hydroxy-2-méthylpropyl)amide (bungeanool), acide 2E,4E,8Z,11E-tétradécatétraénique-N-(2-hydroxy-2-méthylpropyl)amide (isobungeanool), acide 2E,4E,8Z-tétradécatriénique-N-(2-hydroxy-2-méthylpropyl)amide (dihydrobungeanool) et acide 2E,4E-tétradécadiénique-N-(2-hydroxy-2-méthylpropyl)amide (tétrahydrobungeanool);
pour renforcer le début de sucré d'une préparation comprenant:
(ii) l'hespérétine de la formule (I): 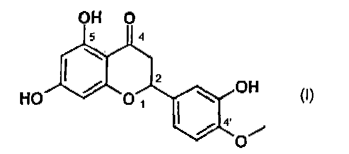 dans laquelle l'hespérétine de la formule (I) existe sous la forme de l'énantiomère (2S), de l'énantiomère (2R) ou d'un mélange quelconque des deux énantiomères et/ou des sels de celle-ci,
et/ou
(iii):
- une 4-hydroxydihydrochalcone de la formule (II): 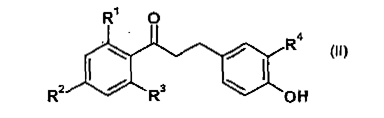
dans laquelle:
R¹, R², R³ et R⁴ signifient indépendamment les uns des autres respectivement H, OH ou un O-alkyle, sous réserve que au moins une des fractions R¹, R² ou R³ signifie OH,
- un sel d'une 4-hydroxydihydrochalcone de la formule (II),
- un mélange comprenant ou composé de deux ou plus 4-hydroxydihydrochalcones différentes de la formule (II), dans laquelle R¹, R², R³ et R⁴ ont respectivement la signification indiquée ci-dessus,
- un mélange comprenant ou composé de sels de deux ou plus 4-hydroxydihydrochalcones différentes de la formule (II), dans laquelle R¹, R², R³ et R⁴ ont respectivement la signification indiquée ci-dessus,
ou
- un mélange comprenant ou composé d'une 4-hydroxydihydrochalcone de la formule (II) ou de deux ou plus 4-hydroxydihydrochalcones différentes de la formule (II), dans laquelle R¹, R², R³ et R⁴ ont respectivement la signification indiquée ci-dessus, et d'un sel d'une 4-hydroxydihydrochalcone de la formule (II) ou de deux sels ou plus de 4-hydroxydihydrochalcones différentes de la formule (II), dans laquelle R¹, R², R³ et R⁴ ont respectivement la signification indiquée ci-dessus,
et comprenant:
(b) une ou plusieurs matières donnant un goût sucré,
et/ou
(c) une ou plusieurs matières aromatisantes, qui provoquent une impression d'odeur sucrée.
